# EUROPEAN PATENT SPECIFICATION

(11) **EP 2 132 319 B1**
(45) Date of publication and mention of the grant of the patent: **01.08.2012**
(21) Application number: 08708940.5
(22) Date of filing: 13.02.2008
(51) Int. Cl.: C12N 15/80, C12P 21/02, C07K 14/435, C12N 9/10

(54) **METHOD FOR PRODUCING AN ANTIFUNGAL PEPTIDE IN A FILAMENTOUS FUNGAL HOST CELL**
VERFAHREN ZUR HERSTELLUNG EINES ANTIPILZPEPTIDS IN EINER FILAMENTÖSEN PILZWIRTSZELLE
METHODE DE PRODUCTION D'UN PEPTIDE ANTIFONGIQUE DANS UNE CELLULE HOTE FONGIQUE, FILAMENTEUSE

(30) Priority: 23.02.2007 DK 200700286
(43) Date of publication of application: 16.12.2009
(73) Proprietor: Novozymes Adenium Biotech A/S, 2880 Bagsvaerd (DK)
(72) Inventor: STEPHENSEN, LÜBECK, Peter, DK-2850 Naerum (DK)
(74) Representative: Jensen, Bo Hammer
(86) International application number: PCT/EP2008/051718
(87) International publication number: WO 2008/101847

(56) References cited:
- WO-A-2004/054366
- US-A1- 2006 211 089
- KURODA M ET AL: "An aureobasidin A resistance gene isolated from Aspergillus is a homolog of yeast AUR1, a gene responsible for inositol phosphorylceramide (IPC) synthase activity." MOLECULAR & GENERAL GENETICS : MGG MAR 1999, vol. 261, no. 2, March 1999 (1999-03), pages 290-296, XP002448097 ISSN: 0026-8925
- WEILER FRANK ET AL: "The Zygosaccharomyces bailii antifungal virus toxin zygocin: Cloning and expression in a heterologous fungal host" MOLECULAR MICROBIOLOGY, vol. 46, no. 4, November 2002 (2002-11), pages 1095-1105, XP002448098 ISSN: 0950-382X
- THEVISSEN KARIN ET AL: "Defensins from insects and plants interact with fungal glucosylceramides." THE JOURNAL OF BIOLOGICAL CHEMISTRY 6 FEB 2004, vol. 279, no. 6, 6 February 2004 (2004-02-06), pages 3900-3905, XP002479767 ISSN: 0021-9258 cited in the application

## Description

### Reference to sequence listing

This application contains a Sequence Listing in computer readable form.

### FIELD OF THE INVENTION

The present invention relates to a method for producing an antifungal peptide in a filamentous fungal host cell and to an *Aspergillus* host cell suitable for producing antifungal peptides.

### BACKGROUND OF THE INVENTION

Filamentous fungi and in particular *Aspergillus* have been widely used for high yield expression of heterologous polypeptides. Production of polypeptides, which are detrimental to the host cell, however, poses a particular problem. Antimicrobial peptides (AMP's) are by nature detrimental to the growth of a wide range of microorganisms dependent on the particular class of AMP in question. One class of small AMP's is the defensins. Defensins are small highly basic cysteine-rich peptides that share a common three-dimensional structure (Thomma et al., (2002) Planta 216: 193-202). Some members of this class of defensins are known to have antifungal activity. These members include dromycin, termicin, and heliomycin. In some yeast like e.g. *Pichia pastoris* and *Candida albicans* it has been demonstrated that gcs-deletion mutants (glucosylceramide synthetase deficient) are resistant to RsAFP2 and heliomycin (Thevissen et al., (2004) Journal of Biological Chemistry 279 (6): 3900-3905). In another yeast like *S. cerevisiae* glucosylceramide has never been detected (Sakaki et al., (2001) Yeast 18: 679-695). In filamentous fungi little is known about the composition of the membrane and only in *Neurospora crassa* has mutants been identified which were resistant to antifungal plant defensins (Ferket et al., (2003) Fungal Genetics and Biology 40: 176-185). In this study analysis of the mutants revealed structurally different GlcCer and altered levels of GlcSte compared to the wild type. However, no conclusion could be made as to whether this change in membrane lipid composition in *N. crassa* mutants correlated to the observed resistance towards plant defensins. The authors speculated that the increased level of GlcSte in the membrane seemed to be linked to the increased resistance. Notably the obtained mutants grew very poorly. The presence of glucosylceramide in other filamentous fungi has been reported in *Aspergillus fumigatus* Boas et al., (1994) Chem. Phys. Lipids 70(1):11-9. It is desirable to isolate mutants of filamentous fungi, particularly *Aspergillus* mutants, which can be used as efficient producers of antifungal peptides, particularly antifungal defensins.

### SUMMARY OF THE INVENTION

The invention provides in a first aspect a method for producing an antifungal peptide in a filamentous fungal host cell, comprising expressing the antifungal peptide in the host cell, wherein an endogenous glucosyl ceramide synthase activity (EC 2.4.1.80) in the filamentous fungal host has been completely or partially inactivated compared to a parent filamentous fungal host grown under identical conditions.

In a second aspect the invention relates to a method for producing an antifungal peptide in a filamentous fungal host cell, comprising expressing the antifungal peptide in the host cell, wherein an endogenous activity encoded by: (a) the nucleotide sequence shown in SEQ ID NO: 1, or (b) a nucleotide sequence having at least 45% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 50% identity with the amino acid sequence of SEQ ID NO: 2, is eliminated or reduced with more than about 60%, preferably more than about 85%, more preferably more than about 90%, and most preferably more than about 95% compared to a parent filamentous fungal host grown under identical conditions.

In a third aspect the invention relates to an *Aspergillus oryzae* host cell, wherein an endogenous activity has been completely or partially inactivated, and wherein the endogenous activity is encoded by: (a) the nucleotide sequence shown in SEQ ID NO: 1, or (b) a nucleotide sequence having at least 60% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 70% identity with the amino acid sequence of SEQ ID NO: 2.

### DEFINITIONS

**Antimicrobial activity:** The term "antimicrobial activity" is defined herein as an activity which is capable of killing or inhibiting growth of microbial cells. In the context of the present invention the term "antimicrobial" is intended to mean that there is a bactericidal and/or a bacteriostatic and/or fungicidal and/or fungistatic effect and/or a virucidal effect, wherein the term "bactericidal" is to be understood as capable of killing bacterial cells. The term "bacteriostatic" is to be understood as capable of inhibiting bacterial growth, i.e. inhibiting growing bacterial cells. The term "fungicidal" is to be understood as capable of killing fungal cells. The term "fungistatic" is to be understood as capable of inhibiting fungal growth, i.e. inhibiting growing fungal cells. The term "virucidal" is to be understood as capable of inactivating virus. The term "microbial cells" denotes bacterial or fungal cells (including yeasts).

In the context of the present invention the term "inhibiting growth of microbial cells" is intended to mean that the cells are in the non-growing state, i.e., that they are not able to propagate. Particularly the antimicrobial activity relevant for the method according to the present invention is antifungal. Antifungal activity according to the invention comprises fungicidal and fungistatic activity.

For purposes of the present invention, antimicrobial activity including antifungal activity may be determined according to the procedure described by Lehrer et al., Journal of Immunological Methods, Vol. 137 (2) pp. 167-174 (1991).

**Defensins:** The defensins of the invention may be any antimicrobial peptide recognized by a person skilled in the art as belonging to the defensin class of antimicrobial peptides. To determine if an antimicrobial peptide is a defensin according to the invention, the amino acid sequence is preferably compared with the hidden markov model profiles (HMM profiles) of the well-known PFAM database (see Example 1).

The defensins may belong to the alpha-defensin class, the beta-defensin class, the theta-defensin class, the insect defensin class, the plant defensin class, the fungal defensin class, the mussel defensin class, or other defensin classes wherein the amino acid sequence comprises 6 or 8 cysteines and are structurally similar to any of the before-mentioned defensin classes. The defensins may also be synthetic defensins sharing the characteristic features of any of the defensin classes. Not all defensins will be antifungal, and therefore the method of the invention will only be relevant in respect of the above defensins, which have antifungal activity.

**cDNA:** The term "cDNA" is defined herein as a DNA molecule which can be prepared by reverse transcription from a mature, spliced, mRNA molecule obtained from a eukaryotic cell. cDNA lacks intron sequences that are usually present in the corresponding genomic DNA. The initial, primary RNA transcript is a precursor to mRNA which is processed through a series of steps before appearing as mature spliced mRNA. These steps include the removal of intron sequences by a process called splicing. cDNA derived from mRNA lacks, therefore, any intron sequences.

**Nucleic acid construct:** The term "nucleic acid construct" as used herein refers to a nucleic acid molecule, either single- or double-stranded, which is isolated from a naturally occurring gene or which is modified to contain segments of nucleic acids in a manner that would not otherwise exist in nature. The term nucleic acid construct is synonymous with the term "expression cassette" when the nucleic acid construct contains the control sequences required for expression of a coding sequence of the present invention.

**Control sequence:** The term "control sequences" is defined herein to include all components, which are necessary or advantageous for the expression of a polynucleotide encoding a polypeptide of the present invention. Each control sequence may be native or foreign to the nucleotide sequence encoding the polypeptide. Such control sequences include, but are not limited to, a leader, polyadenylation sequence, propeptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the nucleotide sequence encoding a polypeptide.

**Operably linked:** The term "operably linked" denotes herein a configuration in which a control sequence is placed at an appropriate position relative to the coding sequence of the polynucleotide sequence such that the control sequence directs the expression of the coding sequence of a polypeptide.

**Coding sequence:** When used herein the term "coding sequence" means a nucleotide sequence, which directly specifies the amino acid sequence of its protein product. The boundaries of the coding sequence are generally determined by an open reading frame, which usually begins with the ATG start codon or alternative start codons such as GTG and TTG. The coding sequence may be a DNA, cDNA, or recombinant nucleotide sequence.

**Expression:** The term "expression" includes any step involved in the production of the polypeptide including, but not limited to, transcription, post-transcriptional modification, translation, post-translational modification, and secretion.

**Expression vector:** The term "expression vector" is defined herein as a linear or circular DNA molecule that comprises a polynucleotide encoding a defensin peptide, and which is operably linked to additional nucleotides that provide for its expression.

**Host cell:** The term "host cell", as used herein, includes any cell type which is susceptible to transformation, transfection, transduction, and the like with a nucleic acid construct of the present invention.

**Modification:** The term "modification" means herein any chemical modification of the defensin. The modification(s) can be substitution(s), deletion(s) and/or insertions(s) of the amino acid(s) as well as replacement(s) of amino acid side chain(s); or use of unnatural amino acids with similar characteristics in the amino acid sequence. In particular the modification(s) can be amidations, such as amidation of the C-terminus.

**Identity:** The relatedness between two amino acid sequences or between two nucleotide sequences is described by the parameter "identity".

For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

The degree of identity between an amino acid sequence of the present invention ("invention sequence"); e.g. amino acids 1 to 517 of SEQ ID NO: 2 and a different amino acid sequence ("foreign sequence") is calculated as the number of exact matches in an alignment of the two sequences, divided by the length of the "invention sequence" or the length of the "foreign sequence", whichever is the shortest. The result is expressed in percent identity.

An exact match occurs when the "invention sequence" and the "foreign sequence" have identical amino acid residues in the same positions of the overlap (in the alignment example below this is represented by "I"). The length of a sequence is the number of amino acid residues in the sequence (e.g. the length of SEQ ID NO:2 is 517).

In the purely hypothetical alignment example below, the overlap is the amino acid sequence "HTWGER-NL" of Sequence 1; or the amino acid sequence "HGWGEDANL" of Sequence 2. In the example a gap is indicated by a "-".

Hypothetical alignment example:

For purposes of the present invention, the degree of identity between two nucleotide sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

In a particular embodiment, the percentage of identity of an amino acid sequence of a polypeptide with, or to, amino acids 1 to 517 of SEQ ID NO: 2 is determined by i) aligning the two amino acid sequences using the Needle program, with the BLOSUM62 substitution matrix, a gap opening penalty of 10, and a gap extension penalty of 0.5; ii) counting the number of exact matches in the alignment; iii) dividing the number of exact matches by the length of the shortest of the two amino acid sequences, and iv) converting the result of the division of iii) into percentage. The percentage of identity to, or with other sequences of the invention, is calculated in an analogous way.

For purposes of the present invention, the degree of identity between two nucleotide sequences is determined by the Wilbur-Lipman method (Wilbur and Lipman, 1983, Proceedings of the National Academy of Science USA 80: 726-730) using the LASERGENE™ MEGALIGN™ software (DNASTAR, Inc., Madison, WI) with an identity table and the following multiple alignment parameters: Gap penalty of 10 and gap length penalty of 10. Pairwise alignment parameters are Ktuple=3, gap penalty=3, and windows=20.

### DETAILED DESCRIPTION OF THE INVENTION

In the present invention it has been discovered that certain mutants of filamentous fungi can be used as host cells for the production of antifungal peptides. The present inventors have found that filamentous fungi, particularly belonging to the genus *Aspergillus,* when deleted for and endogenous gene having the nucleotide sequence as shown in SEQ ID NO: 1, which DNA sequence encodes the enzyme with the amino acid sequence of SEQ ID NO: 2, are capable of producing high amounts of antifungal defensins, such as e.g. the heliomycin derivative ETD-151.

In one embodiment according to the invention the host cell has a reduced expression or no expression of the polypeptide having the amino acid sequence shown in SEQ ID NO: 2 or a functional homologue of SEQ ID NO: 2. Particularly the functional homologue is at least 50 % identical to SEQ ID NO: 2, particularly at least 60 % identical to SEQ ID NO: 2, more particularly at least 70 % identical to SEQ ID NO: 2, particularly at least 80 % identical to SEQ ID NO: 2, particularly at least 85% identical to SEQ ID NO: 2, particularly at least 90%, more particularly at least 95%, most particularly at least 98% identical to SEQ ID NO: 2.

In one embodiment the polynucleotide encoding the polypeptide of SEQ ID NO: 2 is the polynucleotide shown in SEQ ID NO: 1 or any other polynucleotide capable of encoding the polypeptide of SEQ ID NO: 2. The polynucleotide according to the invention could in another embodiment be a polynucleotide encoding a functional homologue of the polypeptide shown in SEQ ID NO: 2.

In another embodiment of the invention the expression of the polypeptide shown in SEQ ID NO: 2 or a homologue thereof has been reduced or eliminated in the filamentous fungal host cell. The polynucleotide encoding the polypeptide may be at least 45 % identical to the polynucleotide having the nucleic acid sequence of SEQ ID NO: 1, particularly at least 50 % identical, more particularly at least 60 % identical, particularly at least 65 % identical, more particularly at least 70 % identical, more particularly at least 75 % identical, more particularly at least 80 % identical, more particularly at least 85 % identical, even more particularly at least 90 % identical, most particularly at least 95 % identical.

In another embodiment the polypeptide having the amino acid sequence of SEQ ID NO: 2 or a functional homologue thereof is encoded by a polynucleotide which hybridizes under at least low stringency conditions with (i) nucleotides 1 to 1554 of SEQ ID NO: 1, or (ii) a complementary strand of (i).

In another embodiment the polypeptide having the amino acid sequence of SEQ ID NO: 2 or a functional homologue thereof is encoded by a polynucleotide which hybridizes under at least medium stringency conditions with (i) nucleotides 1 to 1554 of SEQ ID NO: 1, or (ii) a complementary strand of (i).

In another embodiment the polypeptide having the amino acid sequence of SEQ ID NO: 2 or a functional homologue thereof is encoded by a polynucleotide which hybridizes under at least high stringency conditions with (i) nucleotides 1 to 1554 of SEQ ID NO: 1, or (ii) a complementary strand of (i).

In another embodiment the polypeptide having the amino acid sequence of SEQ ID NO: 2 or a functional homologue thereof is encoded by a polynucleotide which hybridizes under very high stringency conditions with (i) nucleotides 1 to 1554 of SEQ ID NO: 1, or (ii) a complementary strand of (i).

A functional homologue in the context of the present invention means a homologous polypeptide having the same activity, *e.g.* glucosylceramide synthetase activity. Gcs activity can be determined by an enzymatic assay as described in Marks et al., (2001) Journal of Biological Chemistry 276(28): 26492-26498. Alternatively presence of gcs activity can be determined by analysing the composition of the cell membrane as described in Leipelt et al., (2001) Journal of Biological Chemistry, 276 (36): 33621-33629.

In a particular embodiment the host cell is an *Aspergillus* oryzae host cell.

The polypeptide according to the invention the expression of which should be reduced or inactivated may also be a functional homologue of the polypeptide having the amino acid sequence of SEQ ID NO: 2 identified in other filamentous fungal cells. Such functional homologues can be identified starting from the sequence shown in SEQ ID NO: 1, e.g. by using SEQ ID NO: 1 or a fragment thereof, to design a nucleic acid probe to identify and clone DNA encoding polypeptides having gcs activity from strains of different genera or species according to methods well known in the art. In particular, such probes can be used for hybridization with the genomic or cDNA of the genus or species of interest, following standard Southern blotting procedures, in order to identify and isolate the corresponding gene therein. Such probes can be considerably shorter than the entire sequence, but should be at least 14, preferably at least 25, more preferably at least 35, and most preferably at least 70 nucleotides in length. It is, however, preferred that the nucleic acid probe is at least 100 nucleotides in length. For example, the nucleic acid probe may be at least 200 nucleotides, preferably at least 300 nucleotides, more preferably at least 400 nucleotides, or most preferably at least 500 nucleotides in length. Even longer probes may be used, e.g., nucleic acid probes which are at least 600 nucleotides, at least preferably at least 700 nucleotides, more preferably at least 800 nucleotides, or most preferably at least 900 nucleotides in length. Both DNA and RNA probes can be used. The probes are typically labelled for detecting the corresponding gene (for example, with ³²P, ³H, ³⁵S, biotin, or avidin).

A genomic DNA or cDNA library prepared from such other organisms may, therefore, be screened for DNA which hybridizes with the probes described above and which encodes a polypeptide having gcs activity. Genomic or other DNA from such other organisms may be separated by agarose or polyacrylamide gel electrophoresis, or other separation techniques. DNA from the libraries or the separated DNA may be transferred to and immobilized on nitrocellulose or other suitable carrier material. In order to identify a clone or DNA which is homologous with SEQ ID NO: 1 or a subsequence thereof, the carrier material is used in a Southern blot.

For purposes of the present invention, hybridization indicates that the nucleotide sequence hybridizes to a labelled nucleic acid probe corresponding to the nucleotide sequence shown in SEQ ID NO: 1, its complementary strand, or a subsequence thereof, under very low to very high stringency conditions. Molecules to which the nucleic acid probe hybridizes under these conditions can be detected using X-ray film.

For long probes of at least 100 nucleotides in length, very low to very high stringency conditions are defined as prehybridization and hybridization at 42°C in 5X SSPE, 0.3% SDS, 200 µg/ml sheared and denatured salmon sperm DNA, and either 25% formamide for very low and low stringencies, 35% formamide for medium and medium-high stringencies, or 50% formamide for high and very high stringencies, following standard Southern blotting procedures for 12 to 24 hours optimally.

For long probes of at least 100 nucleotides in length, the carrier material is finally washed three times each for 15 minutes using 2X SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

In a particular embodiment, the wash is conducted using 0.2X SSC, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency). In another particular embodiment, the wash is conducted using *0.1X SSC*, 0.2% SDS preferably at least at 45°C (very low stringency), more preferably at least at 50°C (low stringency), more preferably at least at 55°C (medium stringency), more preferably at least at 60°C (medium-high stringency), even more preferably at least at 65°C (high stringency), and most preferably at least at 70°C (very high stringency).

The skilled person will know how to introduce mutations in an identified gene e.g. by deleting part of or the complete coding sequence of the gene. Alternatively mutations can be introduced by random mutagenesis or site directed mutagenesis.

Relevant antifungal peptides according to the invention are all peptides that have the gcs gene product as target.

One particularly relevant group of peptides are defensins. Defensins are small highly basic cysteine-rich peptides that share a common three-dimensional structure (Thomma et al., (2002) Planta 216: 193-202). Some members of this class of defensins are known to have antifungal activity. These members include dromycin, termicin, and heliomycin. Examples of defensins include, but are not limited to, alpha-Defensin HNP-1 (human neutrophil peptide) HNP-2 and HNP-3; beta-Defensin-12, Drosomycin, Heliomicin, gamma1-purothionin, Insect defensin A, and the defensins disclosed in PCT applications WO 99/53053 and WO 02/085934,

Particularly the defensins relevant to the aspect of the present invention have in one embodiment antifungal activity in which case they may be difficult to express in a fungal expression system.

In a particular embodiment the defensin is selected from the group consisting of heliomycin, drosomycin, termicin, HsAFP3, RsAFP1, RsAFP2 or derivatives thereof.

In another embodiment the defensin is a heliomycin derivative selected from the group consisting of ARD1, ETD-135, ETD-151.

A defensin may be obtained from microorganisms of any genus. For purposes of the present invention, the term "obtained from" as used herein in connection with a given source shall mean that the defensin encoded by a nucleotide sequence is produced by the source or by a strain in which the nucleotide sequence from the source has been inserted. In a preferred aspect, the defensin obtained from a given source is secreted extracellularly.

A defensin may be a fungal defensin, and e.g. a yeast defensin such as a *Candida*, *Kluyveromyces*, *Pichia*, *Saccharomyces*, *Schizosaccharomyces*, or *Yarrowia* defensin; or it may be a filamentous fungal defensin such as an *Acremonium*, *Aspergillus*, *Aureobasidium*, *Cryptococcus, Filibasidium, Fusarium, Humicola, Magnaporthe, Mucor, Myceliophthora, Neocallimastix, Neurospora, Paecilomyces, Penicillium, Piromyces, Schizophyllum, Talaromyces, Thermoascus*, *Thielavia*, *Tolypocladium*, or *Trichoderma* defensin.

In another aspect, the defensin is a *Saccharomyces carlsbergensis*, *Saccharomyces cerevisiae, Saccharomyces diastaticus, Saccharomyces douglasii, Saccharomyces kluyveri, Saccharomyces norbensis*, or *Saccharomyces oviformis* defensin having antimicrobial activity.

In another aspect, the defensin is an *Aspergillus aculeatus*, *Aspergillus awamori*, *Aspergillus fumigatus, Aspergillus foetidus, Aspergillus japonicus, Aspergillus nidulans, Aspergillus niger*, *Aspergillus oryzae*, *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium oxysporum*, *Fusarium reticulatum*, *Fusarium roseum*, *Fusarium sambucinum*, *Fusarium sarcochroum*, *Fusarium sporotrichioides*, *Fusarium sulphureum*, *Fusarium torulosum*, *Fusarium trichothecioides*, *Fusarium venenatum*, *Humicola insolens*, *Humicola lanuginosa*, *Mucor miehei*, *Myceliophthora thermophila*, *Neurospora crassa*, *Penicillium purpurogenum*, *Trichoderma harzianum*, *Trichoderma koningii*, *Trichoderma longibrachiatum*, *Trichoderma reesei*, or *Trichoderma viride* defensin.

Strains of these species are readily accessible to the public in a number of culture collections, such as the American Type Culture Collection (ATCC), Deutsche Sammlung von Mikroorganismen und Zellkulturen GmbH (DSM), Centraalbureau Voor Schimmelcultures (CBS), and Agricultural Research Service Patent Culture Collection, Northern Regional Research Center (NRRL).

Defensins also include fused defensins or cleavable fusion defensins in which another defensin is fused at the N-terminus or the C-terminus of the defensin or fragment thereof. A fused defensin is produced by fusing a nucleotide sequence (or a portion thereof) encoding another defensin to a nucleotide sequence (or a portion thereof) of the present invention. Techniques for producing fusion defensins are known in the art, and include ligating the coding sequences encoding the defensins so that they are in frame and that expression of the fused defensin is under control of the same promoter(s) and terminator.

### Nucleic Acid Sequences

The techniques used to isolate or clone a polynucleotide encoding a defensin are known in the art and include isolation from genomic DNA, preparation from cDNA, or a combination thereof. Cloning of the polynucleotides encoding the defensins from such genomic DNA can be effected, *e.g.*, by using the well known polymerase chain reaction (PCR) or antibody screening of expression libraries to detect cloned DNA fragments with shared structural features. See, *e.g.*, Innis et al., 1990, PCR: A Guide to Methods and Application, Academic Press, New York. Other nucleic acid amplification procedures such as ligase chain reaction (LCR), ligated activated transcription (LAT) and nucleotide sequence-based amplification (NASBA) may be used. The polynucleotides may be cloned from a strain of *Eurotium*, *Aspergillus*, *Pseudoplectania*, *Crassostrea*, *Mesobuthus* or another or related organism and thus, for example, may be an allelic or species variant of the defensin encoding region of the nucleotide sequences.

Modification of a nucleotide sequence encoding a defensin may be necessary for the synthesis of defensins substantially similar to the defensin. The term "substantially similar" to the defensin refers to non-naturally occurring forms of the defensin. These defensins may differ in some engineered way from the defensin isolated from its native source, *e.g.*, artificial variants that differ in specific activity, thermostability, pH optimum, or the like. For a general description of nucleotide substitution, see, *e.g.*, Ford et al., 1991, Protein Expression and Purification 2: 95-107.

### Nucleic Acid Constructs

The present invention also relates to nucleic acid constructs comprising a polynucleotide encoding a defensin operably linked to one or more control sequences which direct the expression of the coding sequence in a suitable host cell under conditions compatible with the control sequences.

A polynucleotide encoding a defensin may be manipulated in a variety of ways to provide for expression of the defensin. Manipulation of the polynucleotide's sequence prior to its insertion into a vector may be desirable or necessary depending on the expression vector. The techniques for modifying polynucleotide sequences utilizing recombinant DNA methods are well known in the art.

The control sequence may be an appropriate promoter sequence, a nucleotide sequence which is recognized by a host cell for expression of a polynucleotide encoding a defensin of the invention. The promoter sequence contains transcriptional control sequences which mediate the expression of the defensin. The promoter may be any nucleotide sequence which shows transcriptional activity in the host cell of choice including mutant, truncated, and hybrid promoters, and may be obtained from genes encoding extracellular or intracellular polypeptides either homologous or heterologous to the host cell.

Examples of suitable promoters for directing the transcription of the nucleic acid constructs of the present invention in a filamentous fungal host cell are promoters obtained from the genes for *Aspergillus oryzae* TAKA amylase, *Rhizomucor miehei* aspartic proteinase, *Aspergillus niger* neutral alpha-amylase, *Aspergillus niger* acid stable alpha-amylase, *Aspergillus niger* or *Aspergillus awamori* glucoamylase (*glaA*), *Rhizomucor miehei* lipase, *Aspergillus oryzae* alkaline protease, *Aspergillus oryzae* triose phosphate isomerase, *Aspergillus nidulans* acetamidase, *Fusarium venenatum* amyloglucosidase (WO 00/56900), *Fusarium venenatum* Daria (WO 00/56900), *Fusarium venenatum* Quinn (WO 00/56900), *Fusarium oxysporum* trypsin-like protease (WO 96/00787), *Trichoderma reesei* beta-glucosidase, *Trichoderma reesei* cellobiohydrolase I, *Trichoderma reesei* endoglucanase I, *Trichoderma reesei* endoglucanase II, *Trichoderma reesei* endoglucanase III, *Trichoderma reesei* endoglucanase IV, *Trichoderma reesei* endoglucanase V, *Trichoderma reesei* xylanase I, *Trichoderma reesei* xylanase II, *Trichoderma reesei* beta-xylosidase, as well as the NA2-tpi promoter (a hybrid of the promoters from the genes for *Aspergillus niger* neutral alpha-amylase and *Aspergillus oryzae* triose phosphate isomerase); and mutant, truncated, and hybrid promoters thereof.

The control sequence may also be a suitable transcription terminator sequence, a sequence recognized by a host cell to terminate transcription. The terminator sequence is operably linked to the 3' terminus of the nucleotide sequence encoding the defensin. Any terminator which is functional in the host cell of choice may be used in the present invention.

Preferred terminators for filamentous fungal host cells are obtained from the genes for *Aspergillus oryzae* TAKA amylase, *Aspergillus niger* glucoamylase, *Aspergillus nidulans* anthranilate synthase, *Aspergillus niger* alpha-glucosidase, and *Fusarium oxysporum* trypsin-like protease.

The control sequence may also be a suitable leader sequence, a nontranslated region of an mRNA which is important for translation by the host cell. The leader sequence is operably linked to the 5' terminus of the nucleotide sequence encoding the defensin. Any leader sequence that is functional in the host cell of choice may be used in the present invention.

Preferred leaders for filamentous fungal host cells are obtained from the genes for *Aspergillus oryzae* TAKA amylase and *Aspergillus nidulans* triose phosphate isomerase.

The control sequence may also be a polyadenylation sequence, a sequence operably linked to the 3' terminus of the nucleotide sequence and which, when transcribed, is recognized by the host cell as a signal to add polyadenosine residues to transcribed mRNA. Any polyadenylation sequence which is functional in the host cell of choice may be used in the present invention.

Preferred polyadenylation sequences for filamentous fungal host cells are obtained from the genes for *Aspergillus oryzae* TAKA amylase, *Aspergillus niger* glucoamylase, *Aspergillus nidulans* anthranilate synthase, *Fusarium oxysporum* trypsin-like protease, and *Aspergillus niger* alpha-glucosidase.

The control sequence may also be a signal peptide coding region that codes for an amino acid sequence linked to the amino terminus of a polypeptide and directs the encoded polypeptide into the cell's secretory pathway. The 5' end of the coding sequence of the nucleotide sequence may inherently contain a signal peptide coding region naturally linked in translation reading frame with the segment of the coding region which encodes the secreted defensin. Alternatively, the 5' end of the coding sequence may contain a signal peptide coding region which is foreign to the coding sequence. The foreign signal peptide coding region may be required where the coding sequence does not naturally contain a signal peptide coding region. Alternatively, the foreign signal peptide coding region may simply replace the natural signal peptide coding region in order to enhance secretion of the defensin. However, any signal peptide coding region which directs the expressed defensin into the secretory pathway of a host cell of choice may be used in the present invention.

Effective signal peptide coding regions for filamentous fungal host cells are the signal peptide coding regions obtained from the genes for *Aspergillus oryzae* TAKA amylase, *Aspergillus niger* neutral amylase, *Aspergillus niger* glucoamylase, *Rhizomucor miehei* aspartic proteinase, *Humicola insolens* cellulase, and *Humicola lanuginosa* lipase.

The control sequence may also be a propeptide coding region that codes for an amino acid sequence positioned at the amino terminus of a polypeptide. The resultant polypeptide is known as a proenzyme or propolypeptide (or a zymogen in some cases). A propolypeptide is generally inactive and can be converted to a mature active polypeptide by catalytic or autocatalytic cleavage of the propeptide from the propolypeptide. The propeptide coding region may be obtained from the genes for *Bacillus subtilis* alkaline protease (*aprE*), *Bacillus subtilis* neutral protease (*nprT*), *Saccharomyces cerevisiae* alpha-factor, *Rhizomucor miehei* aspartic proteinase, and *Myceliophthora thermophila* laccase (WO 95/33836).

Where both signal peptide and propeptide regions are present at the amino terminus of a polypeptide, the propeptide region is positioned next to the amino terminus of a polypeptide and the signal peptide region is positioned next to the amino terminus of the propeptide region.

It may also be desirable to add regulatory sequences which allow the regulation of the expression of the defensin relative to the growth of the host cell. Examples of regulatory systems are those which cause the expression of the gene to be turned on or off in response to a chemical or physical stimulus, including the presence of a regulatory compound. Regulatory systems in prokaryotic systems include the *lac*, *tac*, and *trp* operator systems. In yeast, the ADH2 system or GAL1 system may be used. In filamentous fungi, the TAKA alpha-amylase promoter, *Aspergillus niger* glucoamylase promoter, and *Aspergillus oryzae* glucoamylase promoter may be used as regulatory sequences. Other examples of regulatory sequences are those which allow for gene amplification. In eukaryotic systems, these include the dihydrofolate reductase gene which is amplified in the presence of methotrexate, and the metallothionein genes which are amplified with heavy metals. In these cases, the nucleotide sequence encoding the defensin would be operably linked with the regulatory sequence.

### Expression Vectors

The present invention also relates to recombinant expression vectors comprising a polynucleotide encoding a defensin, a promoter, and transcriptional and translational stop signals. The various nucleic acids and control sequences described above may be joined together to produce a recombinant expression vector which may include one or more convenient restriction sites to allow for insertion or substitution of the nucleotide sequence encoding the defensin at such sites. Alternatively, a nucleotide sequence encoding a defensin may be expressed by inserting the nucleotide sequence or a nucleic acid construct comprising the sequence into an appropriate vector for expression. In creating the expression vector, the coding sequence is located in the vector so that the coding sequence is operably linked with the appropriate control sequences for expression.

The recombinant expression vector may be any vector (*e.g.*, a plasmid or virus) which can be conveniently subjected to recombinant DNA procedures and can bring about expression of the nucleotide sequence. The choice of the vector will typically depend on the compatibility of the vector with the host cell into which the vector is to be introduced. The vectors may be linear or closed circular plasmids.

The vector may be an autonomously replicating vector, *i.e.*, a vector which exists as an extrachromosomal entity, the replication of which is independent of chromosomal replication, *e.g.*, a plasmid, an extrachromosomal element, a minichromosome, or an artificial chromosome. The vector may contain any means for assuring self-replication. Alternatively, the vector may be one which, when introduced into the host cell, is integrated into the genome and replicated together with the chromosome(s) into which it has been integrated. Furthermore, a single vector or plasmid or two or more vectors or plasmids which together contain the total DNA to be introduced into the genome of the host cell, or a transposon may be used.

The vectors used for expression of the defensins preferably contain one or more selectable markers which permit easy selection of transformed cells. A selectable marker is a gene the product of which provides for biocide or viral resistance, resistance to heavy metals, prototrophy to auxotrophs, and the like.

Examples of selectable markers for use in a filamentous fungal host cell include, but are not limited to, *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), *sC* (sulfate adenyltransferase), and *trpC* (anthranilate synthase), as well as equivalents thereof. Preferred for use in an *Aspergillus* cell are the *amdS* and *pyrG* genes of *Aspergillus nidulans* or *Aspergillus oryzae* and the *bar* gene of *Streptomyces hygroscopicus.*

The vectors preferably contain an element(s) that permits integration of the vector into the host cell's genome or autonomous replication of the vector in the cell independent of the genome.

For integration into the host cell genome, the vector may rely on the polynucleotide's sequence encoding the defensin or any other element of the vector for integration into the genome by homologous or nonhomologous recombination. Alternatively, the vector may contain additional nucleotide sequences for directing integration by homologous recombination into the genome of the host cell at a precise location(s) in the chromosome(s). To increase the likelihood of integration at a precise location, the integrational elements should preferably contain a sufficient number of nucleic acids, such as 100 to 10,000 base pairs, preferably 400 to 10,000 base pairs, and most preferably 800 to 10,000 base pairs, which have a high degree of identity with the corresponding target sequence to enhance the probability of homologous recombination. The integrational elements may be any sequence that is homologous with the target sequence in the genome of the host cell. Furthermore, the integrational elements may be non-encoding or encoding nucleotide sequences. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication which functions in a cell. The term "origin of replication" or "plasmid replicator" is defined herein as a nucleotide sequence that enables a plasmid or vector to replicate *in vivo.*

Examples of origins of replication useful in a filamentous fungal cell are AMA1 and ANS1 (Gems et al., 1991, Gene 98:61-67; Cullen et al., 1987, Nucleic Acids Research 15: 9163-9175; WO 00/24883). Isolation of the AMA1 gene and construction of plasmids or vectors comprising the gene can be accomplished according to the methods disclosed in WO 00/24883.

More than one copy of a polynucleotide encoding a defensin may be inserted into the host cell to increase production of the gene product. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, *e.g.*, Sambrook *et al.*, 1989, *supra*).

### Filamentous Fungal Host Cells

The host cell (or organism) of the invention is a filamentous fungus including all filamentous forms of the divisions Ascomycota, Basidiomycota, Chytridiomycota and Zygomycota (as defined by Kirk, P.M. et al., In, Ainsworth and Bisby's Dictionary of The Fungi, 9th edition, 2001, CAB International, Wallingford, UK). The filamentous fungi are characterized by a vegetative mycelium composed of chitin and glucan and/or other complex polysaccharides. Vegetative growth is by hyphal elongation. Preferably carbon catabolism is obligately aerobic.

In an embodiment, the filamentous fungal host cell (or organism) belongs to the order *Eurotiales* of the subdivision *Ascomycota*; preferably it more specifically belongs to the *Trichocomaceae* family.

In a more preferred embodiment, the filamentous fungal host cell (or organism) is a cell of a species of, but not limited to, *Acremonium*, *Aspergillus*, *Emericella*, *Eurotium*, *Fusarium*, *Humicola*, *Mucor*, *Myceliophthora*, *Neurospora*, *Penicillium*, *Eupenicillium*, *Thielavia*, *Tolypocladium*, and *Trichoderma* or a teleomorph, anamorph or synonym thereof. In an even more preferred embodiment, the filamentous fungal host cell is an *Aspergillus.* In another even more preferred embodiment, the filamentous fungal host cell is an *Acremonium.* In another even more preferred embodiment, the filamentous fungal host cell is a *Fusarium.* In another even more preferred embodiment, the filamentous fungal host cell is a *Humicola.* In another even more preferred embodiment, the filamentous fungal host cell is a *Mucor.* In another even more preferred embodiment, the filamentous fungal host cell is a *Myceliophthora.* In another even more preferred embodiment, the filamentous fungal host cell is a *Neurospora.* In another even more preferred embodiment, the filamentous fungal host cell is a *Penicillium.* In another even more preferred embodiment, the filamentous fungal host cell is a *Thielavia.* In another even more preferred embodiment, the filamentous fungal host cell is a *Tolypocladium.* In another even more preferred embodiment, the filamentous fungal host cell is a *Trichoderma.* In a most preferred embodiment, the filamentous fungal host cell is an *Aspergillus awamori*, *Aspergillus foetidus*, *Aspergillus fumigatus*, *Aspergillus phoenicis*, *Aspergillus japonicus*, *Aspergillus aculeatus*, *Aspergillus nidulans*, *Aspergillus niger* or *Aspergillus oryzae.* In another preferred embodiment, the filamentous fungal host cell is a *Fusarium* of the section Discolor (also known as the section *Fusarium*). For example, the filamentous fungal host cell may be a *Fusarium bactridioides*, *Fusarium cerealis*, *Fusarium crookwellense*, *Fusarium culmorum*, *Fusarium graminearum*, *Fusarium graminum*, *Fusarium heterosporum*, *Fusarium negundi*, *Fusarium reticulatum*, *Fusarium roseum*, *Fusarium sambucinum*, *Fusarium sarcochroum*, *Fusarium sulphureum*, or *Fusarium trichothecioides.* In another prefered embodiment, the filamentous fungal host cell is a *Fusarium* strain of the section Elegans, *e.g.*, *Fusarium oxysporum.* In another most preferred embodiment, the filamentous fungal host cell is a *Humicola insolens* or *Humicola lanuginosa.* In another most preferred embodiment, the filamentous fungal host cell is a *Mucor miehei.* In another most preferred embodiment, the filamentous fungal host cell is a *Myceliophthora thermophilum.* In another most preferred embodiment, the filamentous fungal host cell is a *Neurospora crassa* cell. In another most preferred embodiment, the filamentous fungal host cell is a *Penicillium purpurogenum* or *Penicillium funiculosum* (WO 00/68401). In another most preferred embodiment, the filamentous fungal host cell is a *Thielavia terrestris.* In another most preferred embodiment, the *Trichoderma* cell is a *Trichoderma harzianum*, *Trichoderma koningii*, *Trichoderma longibrachiatum*, *Trichoderma reesei* or *Trichoderma viride.*

In a particular embodiment the filamentous host cell is *Aspergillus oryzae.*

In a preferred embodiment of the invention the host cell is a protease deficient or protease minus strain.

This may e.g. be the protease deficient strain *Aspergillus oryzae* JaL 125 having the alkaline protease gene named "*alp*" deleted. This strain is described in WO 97/35956 (Novozymes), or EP patent no. 429,490, or the TPAP free host cell, in particular a strain of *Aspergillus niger,* disclosed in WO 96/14404. Further, also host cells, especially *A. niger* or *A. oryzae*, with reduced production of the transcriptional activator (prtT) as described in WO 01/68864 is specifically contemplated according to the invention.

### Methods and Uses

In a first aspect, the invention relates to a method for producing an antifungal peptide in a filamentous fungal host cell, comprising expressing the antifungal peptide in the host cell, wherein an endogenous glucosyl ceramide synthase activity in the filamentous fungal host has been completely or partially inactivated compared to a parent filamentous fungal host grown under identical conditions.

In a particular embodiment the host cell is essentially free of any glucosyl ceramide synthetase activity. Particularly it will be convenient to delete the gene encoding the gcs activity. In this case the host cell is a deletion mutant. The skilled person will know how to make deletion mutants as well as other types of mutants.

One way to test whether a mutant cell has a reduced activity of a gcs gene is by analyzing the lipid composition of the membrane in the mutant compared to the wild type. This can be done essentially as described in Leipelt et al., (2001) Journal of Biological Chemistry 276 (36) 33621-33629.

In particular the level of the endogenous glucosyl ceramide synthase activity is reduced more than about 60%, preferably more than about 85%, more preferably more than about 90%, and most preferably more than about 95% compared to a parent filamentous fungal host grown under identical conditions.

In a second aspect the invention provides a method for producing an antifungal peptide in a filamentous fungal host cell, comprising expressing the antifungal peptide in the host cell, wherein an endogenous activity encoded by: (a) the nucleotide sequence shown in SEQ ID NO: 1, or (b) a nucleotide sequence having at least 45% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 50% identity with the amino acid sequence of SEQ ID NO: 2 is eliminated or reduced with more than about 60%, preferably more than about 85%, more preferably more than about 90%, and most preferably more than about 95% compared to a parent filamentous fungal host grown under identical conditions.

In a third aspect the invention provides an *Aspergillus* host cell, wherein an endogenous activity has been completely or partially inactivated, and wherein the endogenous activity is encoded by: (a) the nucleotide sequence shown in SEQ ID NO: 1, or (b) a nucleotide sequence having at least 60% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 70% identity with the amino acid sequence of SEQ ID NO: 2.

In a preferred embodiment the *Aspergillus* host cell is an *Aspergillus oryzae* host cell.

The present invention is further described by the following examples which should not be construed as limiting the scope of the invention.

### EXAMPLES

### Example 1

### Using the HMM files from the PFAM database to identify a defensin

Sequence analysis using hidden markov model profiles (HMM profiles) may be carried out either online on the Internet or locally on a computer using the well-known HMMER freely available software package. The current version is HMMER 2.3.2 from October 2003.

The HMM profiles may be obtained from the well-known PFAM database. The current version is PFAM 16.0 from November 2004. Both HMMER and PFAM are available for all computer platforms from e.g. Washington University in St. Louis (USA), School of Medicine (http://pfam.wustl.edu and http://hmmer.wustl.edu).

If a query amino acid sequence, or a fragment thereof, belongs to one of the following five PFAM families, the amino acid sequence is a defensin according to the present invention:
- Defensin_beta or "Beta Defensin", accession number: PF00711;
- Defensin_propep or "Defensin propeptide", accession number: PF00879;
- Defensin_1 or "Mammalian defensin", accession number: PF00323;
- Defensin_2 or "Arthropod defensin", accession number: PF01097;
- Gamma-thionin or "Gamma-thionins family", accession number: PF00304.

An amino acid sequence belongs to a PFAM family, according to the present invention, if it generates an E-value which is greater than 0.1, and a score which is larger or equal to zero, when the PFAM database is used online, or when the hmmpfam program (from the HMMER software package) is used locally.

When the sequence analysis is carried out locally using the hmmpfam program, it is necessary to obtain (download) the HMM profiles from the PFAM database. Two profiles exist for each family; **xxx_ls.hmm** for glocal searches, and **xxx_fs.hmm** for local searches ("xxx" is the name of the family). That makes a total of ten profiles for the five families mentioned above. Examples of these profiles are shown in figures 1-10 below.

These ten profiles may be used individually, or joined (appended) into a single profile (using a text editor - the profiles are ASCII files) that could be named e.g. defensin.hmm. A query amino acid sequence can then be evaluated by using the following command line:
hmmpfam -E 0.1 defensin.hmm sequence_file
- wherein "sequence_file" is a file with the query amino acid sequence in any of the formats recognized by the HMMER software package.

If the score is larger or equal to zero (0.0), and the E-value is greater than 0.1, the query amino acid sequence is a defensin according to the present invention.

The PFAM database is further described in Bateman et al. (2004) "The Pfam Protein Families Database", Nucleic Acids Research, Vol. 32 (Database Issue) pp. D138-D141.

### Materials

### Strains

*Aspergillus oryzae* ToC1512 is described in WO 2005/070962 A1, example 11
*Aspergillus oryzae* BECh2 is described in WO 00/39322, example 1

### Genes

pyrG: This gene encodes for orotidine-5'-phosphate decarboxylase, an enzyme involved in the biosynthesis of uridine.
Gcs: This gene encodes UDP-glucose:ceramide glucosyltransferase, an enzyme involved in the final step in the glucosylceramide biosynthetic pathway.

### Plasmids

pDV8 is described in WO2001068864A1
pCR^{®}4Blunt TOPO^{®} from Invitrogen
pJaL554 is described in WO2000050567A1
pJaL1028 is described in example 2
pMT2944 is described in example 5
pPtSL2 is described in example 3
pPtSL3 is described in example 3
pPtSL4 is described in example 5

### Primers

172449 (SEQ ID NO: 9)
172450 (SEQ ID NO: 10)
Gcsupfor-1 (SEQ ID NO: 11)
Gcsuprev-1 (SEQ ID NO: 12)
Gcsdownfor-1 (SEQ ID NO: 13)
Gcsdownrev-1 (SEQ ID NO: 14)
Gcs-for (SEQ ID NO: 15)
Gcs-rev (SEQ ID NO: 16)
pJaL1028-BstZ171 (SEQ ID NO: 17)
pJaL1028-EcoRI (SEQ ID NO: 18)
pJaL1028-HindIII (SEQ ID NO: 19)
pJaL1028-BamHI (SEQ ID NO: 20)

**Table 1**

| **Name of primer** | **Primer sequence** | **Primer target** |
|---|---|---|
| 172449 | 5'-GACGAATTCCGATGAATGTGTGTCCTG-3' | |
| 172450 | 5'-GACGAATTCTCTAGAAGATCTCTCGAGGAGCT | |
| | CAAGCTTCTGTACAGTGACCGGTGACTC-3' | |
| Gcsupfor-1 | 5'-TTTGGATCCATAGATGATAGAAACTCTACA-3' | Gcs terminator region |
| Gcsuprev-1 | 5'-TTTAAGCTTGAAGGAACGAGGGACGA-3' | Gcs terminator region |
| Gcsdownfor-1 | 5'-TTTGTATACGTAATTTCAGCCACATCGA-3' | Gcs promoter region |
| Gcsdownrev-1 | 5'-TTTGAATTCCAGGAGTGTTCTGCTGGA-3' | Gcs promoter region |
| Gcs-for | 5'-CTCGACCTTCCGTCAGGA-3' | Gcs gene |
| Gcs-rev | 5'-CCTCATCGAGCTCAACAGA-3' | Gcs gene |
| pJaL1028-BstZ171 | 5'-ACAGATCAATCCTGATCTTGA-3' | pJaL1028 part near BstZ171 |
| pJaL1028-EcoRI | 5'-TACTCTGATAGCTTGACTATGA-3', | pJaL1028 part near EcoRI |
| pJaL1028-HindIII | 5'-CAGGACCCACTGCAGTCA-3' | pJaL1028 part near HindIII |
| pJaL1028-BamHI | 5'-ATGATTACGCCAAGCTCAGA-3' | pJaL1028 part near BamHI |

Table of Primers used for construction of pPtSL3 and for identification of correct homologous recombination in ToC1512.

### Methods

General methods of PCR, cloning, ligation of nucleotides etc. are well known to a person skilled in the art and may for example be found in "Molecular cloning: A laboratory manual", Sambrook et al. (1989), Cold Spring Harbor, NY; Ausubel, F. M. Et al. (eds.); " Current protocols in Molecular Biology", John Wiley and Sons, (1995); Harwood, C. R., and Cutting, S. M. (eds.); "DNA Cloning: A Practical Approach, Volume I and II", D. N. Glover, ed. (1985); "Oligonucleotide Synthesis", M. J.Gait, ed (1984); Nucleic Acid Hybridization", B. D. Hames & S. J. Higgins, eds (1985); A Practical Guide to Molecular Cloning", B. Perbal (1984).

### DNA hybridization

In short all DNA hybridisations were carried out for 16 hours at 65°C in a standard hybridisation buffer of 10x Denhart's solution, 5x SSC, 0.02 M EDTA, 1% SDS, 0.15 mg/ml polyA RNA and 0.05 mg/ml yeast tRNA. After hybridisation the filters were washed in 2x SSC, 0.1 % SDS at 65°C twice and exposed to X-ray films.

### PCR amplification

All PCR amplifications were performed in volumes of 50 microL. containing 1.75 units of Expand High Fidelity PCR System (Roche), approx. 100 ng DNA, 250 microM of each dNTP, and 10 pmol ot two primers desribed above in Expand High Fidelitybuffer with 1,5 mM MgCl₂. Amplification was carried out in a Perkin-Elmer Cetus DNA Termal 480, and consisted of cycle of 2 minutes at 94°C, followed by 35 cycles of 30 seconds at 94°C, 30 seconds of 55°C, and 1 minute at 72°C, followed by an extra extension of 1 minutes at 72°C.

### Transformation of A. oryzae ToC1512:

A 16-20 hour old culture was harvested in Mira cloth and washed with 0,6 M MgSO₄. The mycelia was transferred to a 100 ml plastic tube containing 10 ml MgSO₄, 10 mM NaH₂PO₄, pH 5.8, 50-100 mg Glucanex. Twelve mg BSA was added and the solution was incubated for 2 hours at 37°C under weak shaking. Protoplasts were havested through Mira cloth and 5 ml ST (0,6 M sorbitol, 100 mM Tris-Cl, pH 7,0) was added as a layer upon the protoplasts. After 15 minutes centrifugation at 2500 rpm the protoplast in the interfase was harvested and transferred to a new tube. Two volume of STC (1,2 M sorbitol, 10 mM Tris-Cl, pH 7,5, 10 Mm CaCl₂) was added followed by 5 minutes centrifugation at 2500 rpm. The protoplasts were washed twice in 5 ml STC and finaly resuspended in STC.

Approx. 4 ug DNA was added to 100 µl of protoplasts followed by addition of 300 ul 60 % PEG 4000. The protoplasts were incubated for 20 minutes at room temp. followed by dilution in 1.2 ml sorbitol and centrifugation for 10 minutes at 2500 rpm. The transformants were resuspended in 100 ul sorbitol and plated on FDU selective medium (5-fluoro-2'-deoxy uridine) and incubated at 37°C.

All transformants were re-isolated twice on FDU selective medium from single sporangia and incubated at 30°C.

### Transformation of A. oryzae BECh2 and PtSL1:

Transformation of PtSL1 was performed as described for ToC1512. The transformants were plated on agar plates with amdS selection. All transformants were reisolated twice on amdS selective medium from single sporangia and incubated at 30°C.

Transformation of BECh2 was performed as described for ToC1512. The transformants were plated on agar plates with amdS selection followed by addition of a layer of topagar after 20 minutes of incubation. All transformants were reisolated twice on amdS selective medium from single sporangia and incubated at 30°C.

### Culture conditions and storage:

All strains were stored on Cove-N-Gly slants or Cove-N slants at 4°C and selected transformants in 20 % glycerol at -80°C.

All strains were cultured in YPM before DNA extraction.

Transformants were cultured in 2, 5 or 10 ml YCM or YPM media in Nunc tubes at 30°C for 6 days before SDS gel electrophoresis or for 6-9 days in 5 L lab-tank fermentors.

Selected strains were subjected to YCM with 0-5815 mg/L ETD151 for 6 days in order to test tolerance to ETD151.

### SDS-page gel electrophoresis:

7.5 µl supernatant samples from the above described cultures were subjected to SDS-gel electrophoresis. The gels were stained with SYPRO Orange Protein Gel Stain (Molecular Probes).

### Bioassay:

ETD151 was diluted to 58 µg/ml in peptide dilution buffer (0,1% BSA, 0,01% Acetic Acid) and further on to 6 µg/ml and 0.6 µg/ml and used as control. Candida utilis was grown on YPD plates, A. oryzae on Cove N medium slants. An inoculum of fungal growth was resuspended in 0.1% Tween to an optical density of approx. 1 McFarland Unit. A NUNC-tube with 15 ml 1/3 PD agarose at approx. 42°C was inoculated with approx. 6 X 10⁶ CFU, mixed thoroughly and poured into a sterile 9 cm square dish (Falcon 351112) on a levelling table and allowed to solidify and set for 30 minutes. Ten µl of each of the peptide dilutions was added to the surface of the plate. The plates were incubated at 30°C (or 37°C as appropriate). The next day, growth was detected either by observing the inhibition zones by oblique illumination (or by adding 3 ml of a 3 mM MTT solution and leaving the plate for 1-2 hours at room temperature). The growth inhibition was seen as clearing zones.

### Example 2

### Construction of plasmid pJaL1028

Two restriction recognition sites for BamHI and BgIII, respectively, was destroyed in pDV8 by digestion with BamHI and subsequently the ends were completely filled in by treatment with Klenow and the four dNTPs. The resulting 6030 bp fragment was re-ligated giving plasmid pJaL504. Then secondly pJaL504 was digested with BgIII and then the ends were completely filled in by treatment with Klenow and the 4 dNTPs. The resulting 6034 bp fragment was re-ligated giving plasmid pJaL504-delta-BgIII. By PCR using the primers 172450 and 172449 a 2522 bp fragment was amplified containing the HSV-tk gene flanked by the A.nidulans gpd promoter and TrpC terminator. This PCR fragment was cloned into the plasmid pCR^{®}4Blunt TOPO^{®} vector resulting in pJaL574.

The A.oryzae pyrG gene from pJaL554 was isolated as a 2403 Stul-EcoRI fragment, wherein the EcoRI site was completely filled in by treatment with Klenow and the 4 dNTPs. The fragment was cloned into the unique Pmel site in pJaL574 resulting in plasmid pJaL1022. Plasmid pJaL1022 was digested with SspB1 and the 8574 bp fragment was isolated and re-ligated, resulting in plasmid pJaL1025. Plasmid pJaL1025 was digested with EcoRI and the 8559 bp fragment was isolate and re-ligated, resulting in plasmid pJaL1027. One of two BamHI site was destroyed by partially digestion with BamHI following treatment with Klenow and the four dNTPs by which the ends were completely filled in. The 8563 bp fragment was re-ligated resulting in plasmid pJaL1028.

The resulting plasmid pJaL1028 is suited for construction of homologous double cross-over disruption plasmids for use in Aspergillus. It contains the A.oryzae pyrG gene and the HSV-tk gene. The pyrG gene is surrounded by unique restriction endonuclease (RE) site for facilitating cloning of DNA flanks. At the 3' end of the pyrG there are four unique RE sites (AcII, HindIII, BamHI and Spel) and at the 5' end of the pyrG are two unique RE sites (Bst1107I and EcoRI). The herpex simplex virus I thymidine kinase (HSV-tk) flanked by fungal expression signal makes the host sensitive to 5-flouro-2-deoxyuridine (FdU). A disrupted strain can be isolated by positive selection for the pyrG gene in a pyrG minus host and deselection of the thymidine kinase gene on FdU. Since the tk gene and the pyrG gene are present in the same DNA fragment selection is for transformants in which a double cross-over event has happed. The system gives fewer transformants pr. Micro gram of DNA than transformation with a common disruption cassette, and the frequency of transformants in which the desired homologous recombination has occurred is much higher.

### Example 3

### Construction of plasmid pPtSL3

A DNA fragment of 1192 bp (SEQ ID NO: 3) containing part of the *A. oryzae* strain ToC1512 gcs gene promoter region was PCR amplified using the specific primers Gcsupfor-1 and Gcsuprev-1. The 5'-end of Gcsupfor-1 was flanked with *Bam*HI and the 5'-end of Gcsuprev-1 was flanked with *Hind*III and therefore the fragment was flanked by *Bam*HI and *Hind*III during PCR amplification. The PCR product was digested with *Bam*HI and *Hind*III and cloned into pJaL1028, digested with the same enzymes resulting in the plasmid pPtSL2. The PCR product was sequenced and verified to be identical to the sequence of the gcs promoter region. A DNA fragment of 1236 bp (SEQ ID NO: 4) containing the *A. oryzae* strain ToC1512 gcs gene terminator region was PCR amplified using the specific primers Gcsdownfor-1 and Gcsdownrev-1. The 5'-end of Gcsdownfor-1 was flanked with *BstZ*1107 and the 5'-end of Gcsdownrev-1 was flanked with *Eco*RI and therefore the fragment was flanked by *BstZ*1107 and *Eco*RI during PCR amplification. The PCR product was digested with *BstZ*1107 and *Eco*RI and cloned into pPtSL2, digested with the same enzymes, resulting in the plasmid pPtSL3. The PCR product was sequenced and verified to be identical to the sequence of the gcs terminator region.

### Example 4

### Construction of a gcs⁻ mutant (PtSL1) from A. oryzae strain ToC1512

In order to develop a gcs mutant in *A. oryzae,* strain ToC1512 was transformed with pPtSL3 linearized with *Bam*HI. The transformation was carried out in order to disrupt the gcs gene during gene replacement with pyrG by homologous recombination. A transformant where only the correct replacement has happened without any other introduction of plasmid DNA in the genome was obtained resulting in the *A.oryzae* strain PtSL1 (amy⁻, alp-, Npl⁻, CPA⁻, KA⁻, AMG⁻, gcs⁻).

All ToC1512 transformants were initially tested by PCR for loss of the gcs gene using the primers Gcs-for and Gcs-rev. gcs negative transformants were tested in PCR for insertion of pyrG in place of gcs using the following primer combinations: pJaL1028-BstZ171 + gcsdownrev-1, pJaL1028-HindIII + gcsupfor-1, pJaL1028-BamHI +gcsuprev-1, pJaL1028-EcoRI + gcsdownfor-1 (Table 1).

Southern blotting was performed as described. Southern blot was carried out to confirm that the transformants were lacking the gcs gene, that the pyrG gene was introduced, and that no other parts of the plasmid were introduced in the genome.

A PCR fragment, obtained by the primers Gcs-for and Gcs-rev, of the gcs gene was used as probe to identify strains that had lost the gcs gene. A DNA fragment covering the gcs terminator region was obtained by digestion of pPtSL3 with *Hind*III and *Bam*HI. This was used as probe to identify strains that had obtained the pyrG gene. The plasmid pJaL1028 from which the pyrG cassette and the gcs promoter and terminator regions were deleted (*Eco*RI/*Bam*HI digested) was used as probe to show whether strains had obtained part(s) of the plasmid by incorrect recombination. ³²P was used to label the probes.

For correct insertion the bands on the Southern blot, (Probe 1, gcs terminator region) should give a *Xho*I band at 4608 bp indicating that the gcs gene is disrupted and the pyrG cassette is introduced. If the band is 2309 bp the gcs gene is not disrupted. The bands in this blot are of the size described. The reason why a fragment covering part of the pyrG gene is not used is because remnants of the pyrG gene are already in the genome, and this could give misleading conclusions. Therefore the Southern blot was carried out to identify correct size of restriction fragments. In another Southern blot, using probe 2 (gcs probe) only strains containing the gcs gene should give a band. This should only be the case for ToC1512. A third Southern blot, (Probe 3) shows if any of the mutants have other parts of the plasmid inserted in the genome. Only strain no. 7 has some remnants of the plasmid that should not be introduced.

From the initial FDU selection plates 59 pyrG positive transformants, of which 12 were fast growing, 40 slow growing and 7 extremely slow growing, were reisolated twice at FDU plates. Of these 1 slow growing (candidate 18) and 6 extremely slow growing (candidates 2, 7, 12, 19, 26, 35a) transformants had lost the gcs gene by homologous recombination where the gcs gene was replaced by the pyrG gene. One of these 7 transformants (candidate 7) also had some remnants of the plasmid inserted identified by PCR, primer combination pJaL1028-BamHI + gcsuprev-1 and Southern blot hybridization. The primer combination pJaL1028-EcoRI + gcsdownfor-1 only gave a band on pPtSL3. This means that only strain 7 of the gcs⁻ mutants has remnants of the plasmid part near the BamHI restriction site and the 6 other gcs⁻ mutants are correct disrupted without any remnants of the plasmid part.

To test the tolerance to ETD151, ToC1512, BECh2 and the 7 gcs⁻ mutants were grown in YCM with different amounts of ETD151 (maximum 5815 mg/L). Only the gcs⁻ mutants grew very well in high concentrations of ETD151, while BECh2 grew badly and Toc1512 did not grow at all. One of the gcs⁻ mutants (no. 18) was hereafter selected and named PtSL1.

### Example 5

### Construction of plasmid pPtSL4:

A HinP1I/ Xhol fragment of 140 bp (shown in SEQ ID NO: 5) containing the synthetic ETD151 gene (shown in SEQ ID NO: 6 and encoding the ETD151 peptide shown in SEQ ID NO: 7) was cloned into the plasmid pMT2944 (SEQ ID NO: 8) digested with *Xho*I and *Ac*/1, resulting in the plasmid pPtSL4. Before introducing the ETD151 gene a DNA fragment was removed from pMT2944 by *Xho*I and *Ac*/1 digestion. pMT2944 is briefly described in patent application US20060272050A1 (or WO 2006/053565). It is an *E.coli*/*Aspergillus* shuttle vector allowing selection for leucine prototrophy in E.coli leuB mutants and also for amdS based selection in *Aspergillus oryzae.* The pMT2944 plasmid also contains a strong promoter and a secretion signal allowing insertion of a nucleotide sequence encoding a peptide to be secreted to the growth medium by the *A.oryzae* host. The cloned part in pPtSL4 was sequenced to verify correct cloning and correct DNA sequence.

### Example 6

### Expression of ETD151 in BECh2:

BECh2 was transformed with plasmid pPtSL4 and subjected to amdS medium. amdS resistant colonies were isolated as spores from single sporangies and subjected to amdS medium twice. From 45 BECh2 transformants, 43 were subjected to a Bioassay as described using *Candida utilis* as target organism and to SDS gel electrophoresis. No clear bands the size of ETD151 could be identified from the SDS gels, but the Bioassay showed clearing zones in the plate assay expression of a functional ETD151 peptide showing concentrations around 1 mg/L.

### Example 7

### Expression of ETD151 in PtSL1:

PtSL1 was transformed with plasmid pPtSL4 and subjected to amdS medium. amdS resistant colonies were isolated as spores from single sporangies and subjected to amdS medium twice. From the transformation of PtSL1, 50 amdS resistant transformants were subjected to SDS gel electrophoresis and Bioassay. Of the 50 transformants 30 synthesized a product corresponding in size to ETD151, and these transformants synthesize varying amounts of ETD151 visualized by SDS and Bioassay. The yield was about 2000% higher than for BECh2. Four of the transformants (no. 3, 14, 17 and 21) were subjected to Lab-tank fermentation for 9 days and the yield of ETD151 from these fermentations were raised dramatically resulting in yields that was around 100000% higher. The experiments show that the gcs⁻ mutant PtSL1 expresses ETD151 in much higher concentrations than BECh2.

## Claims

1. A method for producing an antifungal defensin in a filamentous fungal host cell, comprising expressing the antifungal defensin in the host cell, wherein an endogenous glucosyl ceramide synthase activity in the filamentous fungal host has been completely or partially inactivated compared to a parent filamentous fungal host grown under identical conditions.

2. The method according to claim 1, wherein the level of the endogenous glucosyl ceramide synthase activity is reduced more than about 60%, preferably more than about 85%, more preferably more than about 90%, and most preferably more than about 95% compared to a parent filamentous fungal host grown under identical conditions.

3. The method according to claim 2, wherein the host cell is essentially free of any glucosyl ceramide synthase activity.

4. The method according to claim 3, wherein the host cell is a glucosyl ceramide synthase deletion mutant.

5. A method for producing an antifungal defensin in a filamentous fungal host cell, comprising expressing the antifungal defensin in the host cell, wherein an endogenous glucosyl ceramide synthase activity encoded by: (a) the nucleotide sequence shown in SEQ ID NO: 1, or (b) a nucleotide sequence having at least 45% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 50% identity with the amino acid sequence of SEQ ID NO: 2 is eliminated or reduced with more than about 60%, preferably more than about 85%, more preferably more than about 90%, and most preferably more than about 95% compared to a parent filamentous fungal host grown under identical conditions.

6. The method according to any of the preceding claims, wherein the defensin is selected from the group consisting of heliomycin, drosomycin, termicin, HsAFP3, RsAFP1, RsAFP2 or derivatives thereof.

7. The method according to claim 6, wherein the heliomycin derivative is selected from the group consisting of ARD1, ETD-135, ETD-151.

8. The method according to any of the preceding claims, wherein the filamentous fungal host is selected from the group consisting of the genera *Acremonium*, *Aspergillus*, *Emericella*, *Eurotium*, *Fusarium*, *Humicola*, *Mucor*, *Myceliophthora*, *Neurospora*, *Penicillium*, *Eupenicillium*, *Thielavia*, *Tolypocladium*, and *Trichoderma.*

9. The method according to claim 8, wherein the *Aspergillus* is selected from the group consisting of *Aspergillus awamori*, *Aspergillus phoenicis*, *Aspergillus foetidus*, *Aspergillus fumigatus*, *Aspergillus japonicus*, *Aspergillus aculeatus*, *Aspergillus niger*, *Aspergillus nidulans* or *Aspergillus oryzae.*

10. The method according to claim 9, wherein the host cell is an *Aspergillus oryzae* cell.

11. The method according to claim 9, wherein the host cell is an *Aspergillus niger* cell.

12. An *Aspergillus oryzae* host cell, wherein an endogenous activity has been completely or partially inactivated, and wherein the endogenous activity is encoded by: (a) the nucleotide sequence shown in SEQ ID NO: 1, or (b) a nucleotide sequence having at least 60% identity to SEQ ID NO: 1, or (c) a nucleotide sequence which hybridizes under at least medium stringency conditions with SEQ ID NO: 1, or (d) a nucleotide sequence encoding a polypeptide having an amino acid sequence which has at least 70% identity with the amino acid sequence of SEQ ID NO: 2.

## Patentansprüche

1. Verfahren zum Herstellen eines antifungalen Defensins in einer Wirtszelle eines filamentösen Pilzes, umfassend das Exprimieren des antifungalen Defensins in der Wirtszelle, wobei eine endogene Glucosylceramidsynthaseaktivität in der Wirtszelle eines filamentösen Pilzes verglichen mit einer parentalen Wirtszelle eines filamentösen Pilzes, die unter gleichen Bedingungen wachsen gelassen wird, vollständig oder teilweise inaktiviert worden ist.

2. Verfahren nach Anspruch 1, wobei der Spiegel der endogenen Glucosylceramidsynthaseaktivität um mehr als etwa 60%, vorzugsweise um mehr als etwa 85%, stärker bevorzugt um mehr als etwa 90%, und am stärksten bevorzugt um mehr als etwa 95% verglichen mit einer parentalen Wirtszelle eines filamentösen Pilzes, die unter gleichen Bedingungen wachsen gelassen wird, reduziert ist.

3. Verfahren nach Anspruch 2, wobei die Wirtszelle im Wesentlichen frei von jeglicher Glucosylceramidsynthaseaktivität ist.

4. Verfahren nach Anspruch 3, wobei die Wirtszelle eine Glucosylceramidsynthase-Deletionsmutante ist.

5. Verfahren zum Herstellen eines antifungalen Defensins in einer Wirstzelle eines filamentösen Pilzes, umfassend das Exprimieren des antifungalen Defensins in der Wirtszelle, wobei eine endogene Glucosylceramidsynthaseaktivität, kodiert durch: (a) der in SEQ ID NO: 1 gezeigten Nukleotidsequenz, oder (b) eine Nukleotidsequenz mit mindestens 45% Identität zu SEQ ID NO: 1, oder (c) eine Nukleotidsequenz, die unter mindestens mittleren Stringenzbedingungen mit SEQ ID NO: 1 hybridisiert, oder (d) eine Nukleotidsequenz, die ein Polypeptid kodiert, das eine Aminosäuresequenz aufweist, die mindestens 50% Identität mit der Aminosäuresequenz von SEQ ID NO: 2, um mehr als etwa 60%, vorzugsweise um mehr als etwa 85%, stärker bevorzugt um mehr als etwa 90%, und am stärksten bevorzugt um mehr als etwa 95%, verglichen mit einer parentalen Wirtszelle eines filamentösen Pilzes, die unter gleichen Bedingungen wachsen gelassen wird, eliminiert oder reduziert ist.

6. Verfahren nach einem beliebigen der vorhergehenden Ansprüche, wobei das Defensin ausgewählt ist aus der Gruppe bestehend aus Heliomycin, Drosomycin, Termicin, HsAFP3, RsAFP1, RsAFP2, oder Derivaten davon.

7. Verfahren nach Anspruch 6, wobei das Heliomycinderivat ausgewählt ist aus der Gruppe bestehend aus ARD1, ETD-135, ETD-151.

8. Verfahren nach einem beliebigen der vorhergehenden Ansprüche, wobei die Wirtszelle eines filamentösen Pilzes ausgewählt ist aus der Gruppe, bestehend aus den Gattungen *Acremonium, Aspergillus, Emericella, Eurotium, Fusarium, Humicola, Mucor, Myceliophthora, Neurospora, Penicillium, Eupenicillium, Thielavia, Tolypocladium* und *Trichoderma.*

9. Verfahren nach Anspruch 8, wobei der *Aspergillus* ausgewählt ist aus der Gruppe bestehend aus *Aspergillus awamori, Apergillus phoenicus, Aspergillus foetidus, Aspergillus fumigatus, Aspergillus japonicus, Aspergillus aculeatus, Aspergillus niger, Aspergillus nidulans* oder *Aspergillus oryzae.*

10. Verfahren nach Anspruch 9, wobei die Wirtszelle eine Zelle von *Aspergillus oryzae* ist.

11. Verfahren nach Anspruch 9, wobei die Wirtszelle eine Zelle von *Aspergillus niger* ist.

12. Wirtszelle von *Aspergillus oryzae,* wobei eine endogene Aktivität vollständig oder teilweise inaktiviert worden ist, und wobei die endogene Aktivität kodiert ist durch: (a) der in SEQ ID NO: 1 gezeigten Nukleotidsequenz, oder (b) eine Nukleotidsequenz mit mindestens 60% Identität zu SEQ ID NO: 1, oder (c) eine Nukleotidsequenz, die unter mindestens mittleren Stringenzbedingungen mit SEQ ID NO: 1 hybridisiert, oder (d) eine Nukleotidsequenz, die ein Polypeptid kodiert, das eine Aminosäuresequenz aufweist, die mindestens 70% Identität mit der Aminosäuresequenz von SEQ ID NO: 2 aufweist.

## Revendications

1. Procédé de production d'une défensine antifongique dans une cellule hôte de champignon filamenteux, comprenant l'expression de la défensine antifongique dans la cellule hôte, dans lequel une activité glucosyl-céramide synthase endogène dans l'hôte de champignon filamenteux a été complètement ou partiellement inactivée comparativement à un hôte de champignon filamenteux parent cultivé dans des conditions identiques.

2. Procédé selon la revendication 1, dans lequel le niveau d'activité glucosyl-céramide synthase endogène est réduit de plus d'environ 60 %, de préférence plus d'environ 85 %, de manière davantage préférée plus d'environ 90 % et de manière préférée entre toutes, plus d'environ 95 % comparativement à un hôte de champignon filamenteux parent cultivé dans des conditions identiques.

3. Procédé selon la revendication 2, dans lequel la cellule hôte est pratiquement dépourvue de toute activité glucosyl-céramide synthase.

4. Procédé selon la revendication 3, dans lequel la cellule hôte est un mutant par délétion de la glucosyl-céramide synthase.

5. Procédé de production d'une défensine antifongique dans une cellule hôte de champignons filamenteux, comprenant l'expression de la défensine antifongique dans la cellule hôte, dans lequel une activité glucosyl-céramide synthase endogène codée par : (a) la séquence nucléotidique représentée par SEQ ID NO : 1 ou (b) une séquence nucléotidique présentant au moins 45 % d'identité avec SEQ ID NO : 1 ou (c) une séquence nucléotidique qui s'hybride dans des conditions de stringence au moins moyennes avec SEQ ID NO : 1 ou (d) une séquence nucléotidique codant pour un polypeptide ayant une séquence d'acides aminés qui présente au moins 50 % d'identité avec la séquence d'acides aminés de SEQ ID NO : 2, est éliminée ou réduite de plus d'environ 60 %, de préférence plus d'environ 85 %, de manière davantage préférée plus d'environ 90 % et de manière préférée entre toutes, plus d'environ 95 % comparativement à un hôte de champignon filamenteux parent cultivé dans des conditions identiques.

6. Procédé selon l'une quelconque des revendications précédentes, dans lequel la défensine est choisie dans le groupe consistant en l'héliomycine, la drosomycine, la termicine, HsAFP3, RsAFP1, RsAFP2 ou leurs dérivés.

7. Procédé selon la revendication 6, dans lequel le dérivé d'héliomycine est choisi dans le groupe consistant en ARD1, ETD-135, ETD-151.

8. Procédé selon l'une quelconque des revendications précédentes, dans lequel l'hôte de champignon filamenteux est choisi dans le groupe comprenant les genres *Acremonium, Aspergillus, Emericella, Eurotium, Fusarium, Humicola, Mucor, Myceliophthora, Neurospora, Penicillium, Eupenicillium, Thielavia, Tolypocladium* et *Trichoderma.*

9. Procédé selon la revendication 8, dans lequel *l'Aspergillus* est choisi dans le groupe consistant en *Aspergillus awamori, Aspergillus phoenicis, Aspergillus foetidus, Aspergillus fumigatus, Aspergillus japonicus, Aspergillus aculeatus, Aspergillus niger, Aspergillus nidulans* ou *Aspergillus oryzae.*

10. Procédé selon la revendication 9, dans lequel la cellule hôte est une cellule de *Aspergillus oryzae.*

11. Procédé selon la revendication 9, dans lequel la cellule hôte est une cellule de *Aspergillus niger.*

12. Cellule hôte de *Aspergillus oryzae,* dans laquelle une activité endogène a été complètement ou partiellement inactivée, et dans laquelle l'activité endogène est codée par : (a) la séquence nucléotidique représentée par SEQ ID NO : 1 ou (b) une séquence nucléotidique présentant au moins 60 % d'identité avec SEQ ID NO : 1 ou (c) une séquence nucléotidique qui s'hybride dans des conditions de stringence au moins moyennes avec SEQ ID NO : 1 ou (d) une séquence nucléotidique codant pour un polypeptide ayant une séquence d'acides aminés qui présente au moins 70 % d'identité avec la séquence d'acides aminés de SEQ ID NO : 2.


### SEQUENCE LISTING

<110> Novozymes A/S
   Stephensen Lübeck, Peter
<120> Method for producing an antifungal peptide in a filamentous fungal host cell
<130> 11068.204-WO
<160> 22
<170> PatentIn version 3.4
<210> 1
   <211> 1554
   <212> DNA
   <213> Aspergillus oryzae
<400> 1
   atgcttactg taacacaaac taatgcggtc tcaaacagat ggcgatactt tctccaaaga      60
   ccccggaaat cccactccgc aactgcctcc gacgctcccc atgttaccgc gatccgaccc     120
   gtcaaaggcc tcgagccgaa tctgtatgat tgtctggcct cgaccttccg tcaggactat     180
   cctgccgaca aactgaccgt ctatttttgc gtcgcctctc gaagcgatcc tggctaccca     240
   accctgcaaa agcttgtatc tgattttccc cacgccggtg cacgcatctt catcgaggaa     300
   gaggatcctt tgctccagcc aaatggggaa tgtgtctacg acctgggacc aaatccaaag     360
   attcggaaca tgagccgggc ttatagagag gcgaagggtg atattgtctg gatcatagat     420
   tgcaatgttt gggtaggcaa aggagtctgt gggaggatgg tcgaccggct ttgcggactt     480
   ggtgctactt cgggcaaaaa gtacaaattc gtgcatcatt tgccactcgt ggtggacgtc     540
   actagcggtg ccagttcgac agaggagcaa aatgcactag tggagtctta taccaacggc     600
   gatgcagatt caaacagcga tgtgtcctca gcaccgacta tctccaaaca cgaacagggc     660
   actcttgcaa caggtggagg ccgtctagaa gaactgtttc tttcgtcttc tcatgccaaa     720
   atgtacaccg caatcaacac agtcctcatc gcaccctgca tcgtaggcaa atccaacatg     780
   ttccggcggt cgcacctcga ttatctgaca acaccgtccc ctaccgatcc ccacaagcgc     840
   aaccccggta tcgattactt ttccgacaat atttgcgagg atcatctgat cggggacctt     900
   ctctggaaga atcgcgtccg cgaggagaag gagaaaggcg agcgttttgg caagcatggc     960
   atggtctttg gagatctggc tattcagcct gtcggaaaca tgagcgttgg ggactacatc    1020
   gcacgccggg ttcgttggct acgcgtccgc aagttcaccg ttctcctagc aaccttggtc    1080
   gagccgggca cggagtcctt gctgtgctcg tgttacggtg cctggggagt aaccacgtcc    1140
   ttggctcagt tcttgcagga aaaagggttc tgttgtgcaa cttatatgac cacatggact    1200
   gccttcttcg cctttttctt cctcagcttg gcggtctgga tactgaccga ttggacgctg    1260
   tatatcaagt tacattccgc caaatctgtt gagctcgatg aggacacgcc atcctttgca    1320
   cggcctccgt ctggctcttt aaccagacga acattttttc agtggttctc agcgtggctt    1380
   ggacgcgaat tcttggccct ccccatatgg atctgggcct tctacggtgg cgtaactgtt    1440
   acttggcgca accggcgatt ccgagtcgga ctcgacatga aagttcgaga gattggtagt    1500
   gaaaagacac cccagcctaa ctccaaacaa gccccagtcg gtcaagggaa ctga           1554
<210> 2
   <211> 517
   <212> PRT
   <213> Aspergillus oryzae
<400> 2
   Met Leu Thr Val Thr Gln Thr Asn Ala Val Ser Asn Arg Trp Arg Tyr
   1               5                   10                  15
   Phe Leu Gln Arg Pro Arg Lys Ser His Ser Ala Thr Ala Ser Asp Ala
               20                  25                  30
   Pro His Val Thr Ala Ile Arg Pro Val Lys Gly Leu Glu Pro Asn Leu
           35                  40                  45
   Tyr Asp Cys Leu Ala Ser Thr Phe Arg Gln Asp Tyr Pro Ala Asp Lys
       50                  55                  60
   Leu Thr Val Tyr Phe Cys Val Ala Ser Arg Ser Asp Pro Gly Tyr Pro
   65                  70                  75                  80
   Thr Leu Gln Lys Leu Val Ser Asp Phe Pro His Ala Gly Ala Arg Ile
                   85                  90                  95
   Phe Ile Glu Glu Glu Asp Pro Leu Leu Gln Pro Asn Gly Glu Cys Val
               100                 105                 110
   Tyr Asp Leu Gly Pro Asn Pro Lys Ile Arg Asn Met Ser Arg Ala Tyr
           115                 120                 125
   Arg Glu Ala Lys Gly Asp Ile Val Trp Ile Ile Asp Cys Asn Val Trp
       130                 135                 140
   Val Gly Lys Gly Val Cys Gly Arg Met Val Asp Arg Leu Cys Gly Leu
   145                 150                 155                 160
   Gly Ala Thr Ser Gly Lys Lys Tyr Lys Phe Val His His Leu Pro Leu
                   165                 170                 175
   Val Val Asp Val Thr Ser Gly Ala Ser Ser Thr Glu Glu Gln Asn Ala
               180                 185                 190
   Leu Val Glu Ser Tyr Thr Asn Gly Asp Ala Asp Ser Asn Ser Asp Val
           195                 200                 205
   Ser Ser Ala Pro Thr Ile Ser Lys His Glu Gln Gly Thr Leu Ala Thr
       210                 215                 220
   Gly Gly Gly Arg Leu Glu Glu Leu Phe Leu Ser Ser Ser His Ala Lys
   225                 230                 235                 240
   Met Tyr Thr Ala Ile Asn Thr Val Leu Ile Ala Pro Cys Ile Val Gly
   245 250 255
   Lys Ser Asn Met Phe Arg Arg Ser His Leu Asp Tyr Leu Thr Thr Pro
   260 265 270
   Ser Pro Thr Asp Pro His Lys Arg Asn Pro Gly Ile Asp Tyr Phe Ser
   275 280 285
   Asp Asn Ile Cys Glu Asp His Leu Ile Gly Asp Leu Leu Trp Lys Asn
   290 295 300
   Arg Val Arg Glu Glu Lys Glu Lys Gly Glu Arg Phe Gly Lys His Gly
   305 310 315 320
   Met Val Phe Gly Asp Leu Ala Ile Gln Pro Val Gly Asn Met Ser Val
   325 330 335
   Gly Asp Tyr Ile Ala Arg Arg Val Arg Trp Leu Arg Val Arg Lys Phe
   340 345 350
   Thr Val Leu Leu Ala Thr Leu Val Glu Pro Gly Thr Glu Ser Leu Leu
   355 360 365
   Cys Ser Cys Tyr Gly Ala Trp Gly Val Thr Thr Ser Leu Ala Gln Phe
   370 375 380
   Leu Gln Glu Lys Gly Phe Cys Cys Ala Thr Tyr Met Thr Thr Trp Thr
   385 390 395 400
   Ala Phe Phe Ala Phe Phe Phe Leu Ser Leu Ala Val Trp Ile Leu Thr
   405 410 415
   Asp Trp Thr Leu Tyr Ile Lys Leu His Ser Ala Lys Ser Val Glu Leu
   420 425 430
   Asp Glu Asp Thr Pro Ser Phe Ala Arg Pro Pro Ser Gly Ser Leu Thr
   435 440 445
   Arg Arg Thr Phe Phe Gln Trp Phe Ser Ala Trp Leu Gly Arg Glu Phe
   450 455 460
   Leu Ala Leu Pro Ile Trp Ile Trp Ala Phe Tyr Gly Gly Val Thr Val
   465 470 475 480
   Thr Trp Arg Asn Arg Arg Phe Arg Val Gly Leu Asp Met Lys Val Arg
   485 490 495
   Glu Ile Gly Ser Glu Lys Thr Pro Gln Pro Asn Ser Lys Gln Ala Pro
   500 505 510
   Val Gly Gln Gly Asn
   515
<210> 3
   <211> 1192
   <212> DNA
   <213> Aspergillus oryzae
<400> 3
   gtaatttcag ccacatcgaa caagctctct catgtttcga gtttgtttta catagcatag     60
   cagtcccgat atgactgcgt gaaatttgag aatagatatt cagaagcgtc aaaatcaaca    120
   cgaaaccaat tttataatta cacgtttcaa ggaattgaat tgatcgcctc aagtattcag    180
   tccatgtggc ccgtccaccg tcatttatcg gttccacgga tctcatcttc cagtttaccg    240
   gatagcgata gacccaataa tagtatcaag aagacaaatg tccaaagacc aatcgctgtt    300
   ttgaatatac cttttaatgg ccttgttttc cctaaataga agaacccaaa tcgctgaaat    360
   gtcccataat cccgattaca actccaatta atgcgaaagg catctgctat ctgagctagg    420
   ccttcttctt cttccggatc aagtttgctt gaagcacatt gactccgggt gattcatttg    480
   tccctgttgt actagcctct gctgttactg cagttgagac tgtaccgttc ccttcacacc    540
   cttccttttg agctgatgtt gctgttgtcg cttggcttgg ttcatctttc tttcgctttc    600
   gaaccaatcc agtgttgatg atgttggtag ttggagcatg tttatcggca gactctccag    660
   acgtgttcct tcctgtcacc tgctgaccga cccagagctg gtccacgatg gatgcgactt    720
   tctcactcca tgccttggtg ctggctatag aaactttttc gttgtagaag tcttgctcaa    780
   gtaggaaatt tatcgcagtt ttgtaatctt cttcactcat gacagcatgt ccgcggcctg    840
   cttctggcca atagaatgcg ttctggtctg tgttcggctg tactttcgga tgagtctcag    900
   aagataagct gcctgtctgg gttgatgggt cttgtacagg accacctgac gttgcctctg    960
   ctgtagaatc tttggcagtg tccgaagact gggaatcagt agaaacagac accgctgtcc   1020
   ctgtaggttt tgaatccacc ttgtcatata cagggacggt ggttgtcaag gaaccctctg   1080
   ctttctcttt gaaaacaggt ctagagcttg tcatttgaac cgagttgaca agccccggag   1140
   gattaattac aagccacaag aatcctacca gtcgtccagc agaacactcc tg           1192
<210> 4
   <211> 1236
   <212> DNA
   <213> Aspergillus oryzae
<400> 4
   atagatgata gaaactctac aaaaggtgca agccgcattt tgacttcgag atcgaatttc     60
   catcattatc taccatctta tctccctttt tcaattattt tttcaataaa tctttaatat    120
   gacatctgtt gggcatcaaa tacatataaa cctctataac atcccacccc accatcgcca    180
   tcaaaaacca caccatccag aagaaaccac taacaaaccc ccagataccc aaaaatccca    240
   aattcaagag tcacacagac tttgaacaaa ctcgtcacaa gcacgtgatc tcaaaagcgt    300
   caagagacct agacagagag gcaaaaatgt ccacaaagac ccctatcaca ttaaccgtcg    360
   acgcaatcga cgacctcatt tacgacgccc gcgccggcga tctcgaggcc ctcaagtccg 420
   acctggcggc cttgagcgcc cagcacagct gcccgcaggc ctggatcgta gcgtcggcga 480
   tcgactccga gcctgaagag gagggtggtt cgggatcgtg ccttttgcat tttcctgcgg 540
   cgaatgggaa tgagggtatg tacttccttg tctctttttc tctctctttt tttttcttct 600
   ggagagttaa ggttgtttgt tttggggagg gtttcaggga gtggatgttt aggattagat 660
   gggaaaggct aattgggtat ttcagaaatc ttgaacttcc tcctgggagt gctcactcag 720
   ggtgagactc agctggatca ggctcaggtt gcggctgtta ttaaccaccg taatcactcg 780
   gggaacacgg cgttgcactg ggcggcgctg aatacgcatc tggagtgcgt gaaggcgctt 840
   gttggggctg gtgctgatat ctccgttaag aatgatgctg ggttagatgc tgttttcctt 900
   gctgagaggg ctgattggtc gactgaggag cagggcgagg agcaggatga ggtcgaggtt 960
   gaaatagagg ctgaggctga ggcacagaag gttcaaggcg atgctgggaa gatgtctaag 1020
   gggagacagg tcgcggagtg gttgctgggt tcggagaagg gtggtgaatt ggaaactggt 1080
   gctggggaga atgctcctgc tgctgcggag gtgtcgacgc aatgatttac gctgtttatt 1140
   gtttttatag atctgttgga attggataga aaagttcttt atattttttt aacgatttca 1200
   ttcagtgata ttcgtacatt cgtccctcgt tccttc 1236
<210> 5
   <211> 153
   <212> DNA
   <213> Artificial
<220>
<223> Codon optimized sequence
<400> 5
   caaagcgcga taaactcatt ggttcgtgtg tctggggtgc agtgaactac acttccaact 60
   gtcgagccga gtgtaagcgt aggggctaca aaggtggaca ctgtggttcc ttcgcaaacg 120
   tcaactgttg gtgtgaaacc taactcgagt ctc 153
<210> 6
   <211> 132
   <212> DNA
   <213> Artificial
<220>
<223> Codon optimized sequence
<400> 6
   gataaactca ttggttcgtg tgtctggggt gcagtgaact acacttccaa ctgtcgagcc 60
   gagtgtaagc gtaggggcta caaaggtgga cactgtggtt ccttcgcaaa cgtcaactgt 120
   tggtgtgaaa cc 132
<210> 7
   <211> 44
   <212> PRT
   <213> Artificial
<220>
<223> heliomycin derivative
<400> 7
   Asp Lys Leu Ile Gly Ser Cys Val Trp Gly Ala Val Asn Tyr Thr Ser
   1               5                   10                  15
   Asn Cys Arg Ala Glu Cys Lys Arg Arg Gly Tyr Lys Gly Gly His Cys
               20                  25                  30
   Gly Ser Phe Ala Asn Val Asn Cys Trp Cys Glu Thr
           35                  40
<210> 8
   <211> 7288
   <212> DNA
   <213> Artificial
<220>
<223> Complete sequence of expression plasmid
<400> 8
   tgtataagct agcttatggt gttttgatca ttttaaattt ttatatggcg ggtggtgggc    60
   aactcgcttg cgcgggcaac tcgcttaccg attacgttag ggctgatatt tacgtaaaaa   120
   tcgtcaaggg atgcaagacc aaaccgttaa atttccggag tcaacagcat ccaagcccaa   180
   gtccttcacg gagaaacccc agcgtccaca tcacgagcga aggaccacct ctaggcatcg   240
   gacgcaccat ccaattagaa gcagcaaagc gaaacagccc aagaaaaagg tcggcccgtc   300
   ggccttttct gcaacgctga tcacgggcag cgatccaacc aacaccctcc agagtgacta   360
   ggggcggaaa tttatcggga ttaatttcca ctcaaccaca aatcacagtc gtccccggta   420
   atttaacggc tgcagacggc aatttaacgg cttctgcgaa tcgcttggat tccccgcccc   480
   tggccgtaga gcttaaagta tgtcccttgt cgatgcgatg tatgaattca tggtgttttg   540
   atcattttaa atttttatat ggcgggtggt gggcaactcg cttgcgcggg caactcgctt   600
   accgattacg ttagggctga tatttacgta aaaatcgtca agggatgcaa gaccaaaccg   660
   ttaaatttcc ggagtcaaca gcatccaagc ccaagtcctt cacggagaaa ccccagcgtc   720
   cacatcacga gcgaaggacc acctctaggc atcggacgca ccatccaatt agaagcagca   780
   aagcgaaaca gcccaagaaa aaggtcggcc cgtcggcctt ttctgcaacg ctgatcacgg   840
   gcagcgatcc aaccaacacc ctccagagtg actaggggcg gaaatttatc gggattaatt   900
   tccactcaac cacaaatcac agtcgtcccc ggtaatttaa cggctgcaga cggcaattta   960
   acggcttctg cgaatcgctt ggattccccg cccctggccg tagagcttaa agtatgtccc  1020
   ttgtcgatgc gatgtatcac aacatataaa tactggcaag ggatgccatg cttggagttt  1080
   ccaactcaat ttacctctat ccacacttct cttccttcct caatcctcta tatacacaac  1140
   tggggatcca ccatgcaatt taccaccatc ctctccatcg gtatcaccgt cttcggactg  1200
   ggtatgtaca ccaccccctt gcgtctgatc tgtgacatat gtagctgact ggtcagccaa  1260
   caccggagcc tttgcagcac cccagccggt acccgaggct tacgctgttt ctgatcccga  1320
   ggctcatcct gacgattttg ctggtatgga tgcgaaccaa cttcagaaac gttgatttgg   1380
   atgcaatggt ccttgggatg aggatgatat gcagtgccac aatcactgca agtctattaa   1440
   gggttacaag ggaggttatt gtgctaaggg gggctttgtt tgcaagtgtt actagctcga   1500
   gatctagagg gtgactgaca cctggcggta gacaatcaat ccatttcgct atagttaaag   1560
   gatggggatg agggcaattg gttatatgat catgtatgta gtgggtgtgc ataatagtag   1620
   tgaaatggaa gccaagtcat gtgattgtaa tcgaccgacg gaattgagga tatccggaaa   1680
   tacagacacc gtgaaagcca tggtctttcc ttcgtgtaga agaccagaca gacagtccct   1740
   gatttaccct tgcacaaagc actagaaaat tagcattcca tccttctctg cttgctctgc   1800
   tgatatcact gtcattcaat gcatagccat gagctcatct tagatccaag cacgtaattc   1860
   catagccgag gtccacagtg gagcagcaac attccccatc attgctttcc ccaggggcct   1920
   cccaacgact aaatcaagag tatatctcta ccgtccaata gatcgtcttc gcttcaaaat   1980
   ctttgacaat tccaagaggg tccccatcca tcaaacccag ttcaataata gccgagatgc   2040
   atggtggagt caattaggca gtattgctgg aatgtcgggg ccagttggcc gggtggtcat   2100
   tggccgcctg tgatgccatc tgccactaaa tccgatcatt gatccaccgc ccacgaggcg   2160
   cgtctttgct ttttgcgcgg cgtccaggtt caactctctc ctctagactg gaaacgcaac   2220
   cctgaaggga ttcttccttt gagagatgga agcgtgtcat atctcttcgg ttctacggca   2280
   ggtttttttc tgctctttcg tagcatggca tggtcacttc agcgcttatt tacagttgct   2340
   ggtattgatt tcttgtgcaa attgctatct gacacttatt agctatggag tcaccacatt   2400
   tcccagcaac ttccccactt cctctgcaat cgccaacgtc ctctcttcac tgagtctccg   2460
   tccgataacc tgcactgcaa ccggtgcccc atggtacgcc tccggatcat actcttcctg   2520
   cacgagggca tcaagctcac taaccgcctt gaaactctca ttcttcttat cgatgttctt   2580
   atccgcaaag gtaaccggaa caaccacgct cgtgaaatcc agcaggttga tcacagaggc   2640
   atacccatag taccggaact ggtcatgccg taccgcagcg gtaggcgtaa tcggcgcgat   2700
   gatggcgtcc agttccttcc cggccttttc ttcagcctcc cgccatttct caaggtactc   2760
   catctggtaa ttccacttct ggagatgcgt gtcccagagc tcgttcatgt taacagcttt   2820
   gatgttcggg ttcagtaggt ctttgatatt tggaatcgcc ggctcgccgg atgcactgat   2880
   atcgcgcatt acgtcggcgc tgccgtcagc cgcgtagata tgggagatga gatcgtggcc   2940
   gaaatcgtgc ttgtatggcg tccacggggt cacggtgtga ccggctttgg cgagtgcggc   3000
   gacggtggtt tccacgccgc gcaggatagg agggtgtgga aggacattgc cgtcgaagtt   3060
   gtagtagccg atattgagcc cgccgttctt gatcttggag gcaataatgt ccgactcgga   3120
   ctggcgccag ggcatgggga tgaccttgga gtcgtatttc catggctcct gaccgaggac   3180
   ggatttggtg aagaggcgga ggtctaacat acttcatcag tgactgccgg tctcgtatat   3240
   agtataaaaa gcaagaaagg aggacagtgg aggcctggta tagagcagga aaagaaggaa   3300
   gaggcgaagg actcaccctc aacagagtgc gtaatcggcc cgacaacgct gtgcaccgtc   3360
   tcctgaccct ccatgctgtt cgccatcttt gcatacggca gccgcccatg actcggcctt 3420
   agaccgtaca ggaagttgaa cgcggccggc actcgaatcg agccaccgat atccgttcct 3480
   acaccgatga cgccaccacg aatcccaacg atcgcaccct caccaccaga actgccgccg 3540
   cacgaccagt tcttgttgcg tgggttgacg gtgcgcccga tgatgttgtt gactgtctcg 3600
   cagaccatca gggtctgcgg gacagaggtc ttgacgtaga agacggcacc ggctttgcgg 3660
   agcatggttg tcagaaccga gtccccttcg tcgtacttgt ttagccatga gatgtagccc 3720
   attgatgttt cgtagccctg gtggcatatg ttagctgaca aaaagggaca tctaacgact 3780
   taggggcaac ggtgtacctt gactcgaagc tggtctttga gagagatggg gaggccatgg 3840
   agtggaccaa cgggtctctt gtgctttgcg tagtattcat cgagttccct tgcctgcgcg 3900
   agagcggcgt cagggaagaa ctcgtgggcg cagtttgtct gcacagaagc cagcgtcagc 3960
   ttgatagtcc cataaggtgg cgttgttaca tctccctgag aggtagaggg gaccctacta 4020
   actgctgggc gattgctgcc cgtttacaga atgctagcgt aacttccacc gaggtcaact 4080
   ctccggccgc cagcttggac acaagatctg cagcggaggc ctctgtgatc ttcagttcgg 4140
   cctctgaaag gatcaccgat ttctttggga aatcaataac gctgtcttcc gcaggcagcg 4200
   tctggacttt ccattcatca gggatggttt ttgcgaggcg ggcgcgctta tcagcggcca 4260
   gttcttccca ggattgaggc attctgtgtt agcttatagt caggatgttg gctcgacgag 4320
   tgtaaactgg gagttggcat gagggttatg taggcttctt tagccccgca tccccctcat 4380
   tctcctcatt gatcccgggg gagcggatgg tgttgataag agactaatta tagggtttag 4440
   ctggtgccta gctggtgatt ggctggcttc gccgaatttt acgggccaag gaaagctgca 4500
   gaaccgcggc actggtaaac ggtaattaag ctatcagccc catgctaacg agtttaaatt 4560
   acgtgtattg ctgataaaca ccaacagagc tttactgaaa gatgggagtc acggtgtggc 4620
   ttccccactg cgattattgc acaagcagcg agggcgaact tgactgtcgt cgctgagcag 4680
   cctgcagtca aacatacata tatatcaacc gcgaagacgt ctggccttgt agaacacgac 4740
   gctccctagc aacacctgcc gtgtcagcct ctacggttgt tacttgcatt caggatgctc 4800
   tccagcgggc gagctattca aaatattcaa agcaggtatc tcgtattgcc aggattcagc 4860
   tgaagcaaca ggtgccaagg aaatctgcgt cggttctcat ctgggcttgc tcggtcctgg 4920
   cgtagatcta gagtcgacct gcaggcatgc ggcgtaatca tggtcatagc tgtttcctgt 4980
   gtgaaattgt tatccgctca caattccaca caacatacga gccggaagca taaagtgtaa 5040
   agcctggggt gcctaatgag tgagctaact cacattaatt gcgttgcgct cactgcccgc 5100
   tttccagtcg ggaaacctgt cgtgccagct gcattaatga atcggccaac gcgcggggag 5160
   aggcggtttg cgtattgggc gctcttccgc ttcctcgctc actgactcgc tgcgctcggt 5220
   cgttcggctg cggcgagcgg tatcagctca ctcaaaggcg gtaatacggt tatccacaga 5280
   atcaggggat aacgcaggaa agaacatgtg agcaaaaggc cagcaaaagg ccaggaaccg 5340
   taaaaaggcc gcgttgctgg cgtttttcca taggctccgc ccccctgacg agcatcacaa 5400
   aaatcgacgc tcaagtcaga ggtggcgaaa cccgacagga ctataaagat accaggcgtt 5460
   tccccctgga agctccctcg tgcgctctcc tgttccgacc ctgccgctta ccggatacct 5520
   gtccgccttt ctcccttcgg gaagcgtggc gctttctcat agctcacgct gtaggtatct 5580
   cagttcggtg taggtcgttc gctccaagct gggctgtgtg cacgaacccc ccgttcagcc 5640
   cgaccgctgc gccttatccg gtaactatcg tcttgagtcc aacccggtaa gacacgactt 5700
   atcgccactg gcagcagcca ctggtaacag gattagcaga gcgaggtatg taggcggtgc 5760
   tacagagttc ttgaagtggt ggcctaacta cggctacact agaagaacag tatttggtat 5820
   ctgcgctctg ctgaagccag ttaccttcgg aaaaagagtt ggtagctctt gatccgacaa 5880
   acaaaccacc gctggtagcg gtggtttttt tgtttgcaag cagcagatta cgcgcagaaa 5940
   aaaaggatct caagaagatc ctttgatctt ttctacgggg tctgacgctc agtggaacga 6000
   aaactcacgt taagggattt tggtcatgag attatcaaaa aggatcttca cctagatcct 6060
   tttaaattaa aaatgaagtt ttaaatcaat ctaaagtata tatgagtaaa cttggtctga 6120
   cagttaagca aggattttct taacttcttc ggcgacagca tcaccgactt cggtggtact 6180
   gttggaacca cctaaatcac cagttctgat acctgcatcc aaaacctttt taactgcatc 6240
   ttcaatggcc ttaccttctt caggcaagtt caatgacaat ttcaacatca ttgcagcaga 6300
   caagatagtg gcgatagggt tgaccttatt ctttggcaaa tctggagcag aaccgtggca 6360
   tggttcgtac aaaccaaatg cggtgttctt gtctggcaaa gaggccaagg acgcagatgg 6420
   caacaaaccc aaggaacctg ggataacgga ggcttcatcg gagatgatat caccaaacat 6480
   gttgctggtg attataatac catttaggtg ggttgggttc ttaactagga tcatggcggc 6540
   agaatcaatc aattgatgtt gaaccttcaa tgtagggaat tcgttcttga tggtttcctc 6600
   cacagttttt ctccataatc ttgaagaggc caaaacatta gctttatcca aggaccaaat 6660
   aggcaatggt ggctcatgtt gtagggccat gaaagcggcc attcttgtga ttctttgcac 6720
   ttctggaacg gtgtattgtt cactatccca agcgacacca tcaccatcgt cttcctttct 6780
   cttaccaaag taaatacctc ccactaattc tctgacaaca acgaagtcag tacctttagc 6840
   aaattgtggc ttgattggag ataagtctaa aagagagtcg gatgcaaagt tacatggtct 6900
   taagttggcg tacaattgaa gttctttacg gatttttagt aaaccttgtt caggtctaac 6960
   actgccggta ccccatttag gaccacccac agcacctaac aaaacggcat cagccttctt 7020
   ggaggcttcc agcgcctcat ctggaagtgg aacacctgta gcatcgatag cagcaccacc 7080
   aattaaatga ttttcgaaat cgaacttgac attggaacga acatcagaaa tagctttaag 7140
   aaccttaatg gcttcggctg tgatttcttg accaacgtgg tcacctggca aaacgacgat 7200
   cttcttaggg gcagacatac tcttcctttt tcaatattat tgaagcattt atcagggtta 7260
   ttgtctcatg agcggataca tatttgaa 7288
<210> 9
   <211> 27
   <212> DNA
   <213> Artificial
<220>
<223> PCR primer
<400> 9
   gacgaattcc gatgaatgtg tgtcctg 27
<210> 10
   <211> 60
   <212> DNA
   <213> Artificial
<220>
<223> PCR primer
<400> 10
   gacgaattct ctagaagatc tctcgaggag ctcaagcttc tgtacagtga ccggtgactc 60
<210> 11
   <211> 30
   <212> DNA
   <213> Artificial
<220>
   <223> PCR primer
<400> 11
   tttggatcca tagatgatag aaactctaca 30
<210> 12
   <211> 26
   <212> DNA
   <213> Artificial
<220>
<223> PCR primer
<400> 12
   tttaagcttg aaggaacgag ggacga 26
<210> 13
   <211> 28
   <212> DNA
   <213> Artificial
<220>
   <223> PCR primer
<400> 13
   tttgtatacg taatttcagc cacatcga 28
<210> 14
   <211> 27
   <212> DNA
   <213> Artificial
<220>
   <223> PCR primer
<400> 14
   tttgaattcc aggagtgttc tgctgga 27
<210> 15
   <211> 18
   <212> DNA
   <213> Artificial
<220>
   <223> PCR primer
<400> 15
   ctcgaccttc cgtcagga 18
<210> 16
   <211> 19
   <212> DNA
   <213> Artificial
<220>
<223> PCR primer
<400> 16
   cctcatcgag ctcaacaga 19
<210> 17
   <211> 21
   <212> DNA
   <213> Artificial
<220>
<223> PCR primer
<400> 17
   acagatcaat cctgatcttg a 21
<210> 18
   <211> 22
   <212> DNA
   <213> Artificial
<220>
<223> PCR primer
<400> 18
   tactctgata gcttgactat ga 22
<210> 19
   <211> 18
   <212> DNA
   <213> Artificial
<220>
<223> PCR primer
<400> 19
   caggacccac tgcagtca 18
<210> 20
   <211> 20
   <212> DNA
   <213> Artificial
<220>
   <223> PCR primer
<400> 20
   atgattacgc caagctcaga 20
<210> 21
   <211> 12
   <212> PRT
   <213> Artificial
<220>
<223> for illustrative purpose
<400> 21
   Ala Cys Met Ser His Thr Trp Gly Glu Arg Asn Leu
   1 5 10
<210> 22
   <211> 14
   <212> PRT
   <213> Artificial
<220>
<223> for illustrative purpose
<400> 22
   His Gly Trp Gly Glu Asp Ala Asn Leu Ala Met Asn Pro Ser
   1 5 10